# EUROPEAN PATENT APPLICATION

(11) **EP 4 653 389 A2**
(43) Date of publication of application: **26.11.2025**
(21) Application number: 25207365.5
(22) Date of filing: 06.09.2019
(51) Int. Cl.: B82Y 30/00

(54) **DIGITAL MICROFLUIDIC (DMF) CARTRIDGE FOR ANALYSIS OF ANALYTES**

(30) Priority: 06.09.2018 US 201862727934 P; 29.05.2019 US 201962854103 P
(62) Divisional of application: 19857917.9
(71) Applicant: Nicoya Lifesciences Inc., Kitchener, Ontario N2G 2K4 (CA)
(72) Inventor: DENOMME, Ryan, Ontario, N2N 3M4 (CA); SUDARSAN, Arjun, Carlsbad, 92009 (US)
(74) Representative: Murgitroyd & Company

(57) **Abstract**

A plasmon resonance (PR) system and instrument, digital microfluidic (DMF) cartridge, and methods of using localized surface plasmon resonance (LSPR) and droplet operations for analysis of analytes is disclosed.

## Description

### CROSS-REFERENCE TO RELATED APPLICATIONS

The presently disclosed subject matter is related to and claims benefit of priority to U.S. Provisional Patent App. No. 62/727,934, entitled "PLASMON RESONANCE (PR) SYSTEM AND INSTRUMENT, DIGITAL MICROFLUIDIC (DMF) CARTRIDGE, AND METHODS OF USING LOCALIZED SURFACE PLASMON RESONANCE (LSPR) FOR ANALYSIS OF ANALYTES" filed on September 6, 2018 and U.S. Provisional Patent App. No. 62/854,103, entitled "PLASMON RESONANCE (PR) SYSTEM AND INSTRUMENT, DIGITAL MICROFLUIDIC (DMF) CARTRIDGE, AND METHODS OF USING LOCALIZED SURFACE PLASMON RESONANCE (LSPR) FOR ANALYSIS OF ANALYTES" filed on May 29, 2019, the entire disclosures of which is incorporated herein by reference.

### TECHNICAL FIELD

The presently disclosed subject matter generally relates to the detection of molecules, such as DNA, proteins, drugs, and the like, and more particularly to a plasmon resonance (PR) system and instrument, digital microfluidic (DMF) cartridge, and methods of using localized surface plasmon resonance (LSPR) and droplet operations for analysis of analytes.

### BACKGROUND

In traditional assays, the protein or DNA arrays are flooded with a solution containing labeled target biomolecules, incubated overnight, rinsed, and then "read-out" using fluorescence detection methods. This is not only time-consuming but requires high sample concentrations. Direct, label-free detection techniques exist, such as surface plasmon resonance (SPR). However, these techniques exhibit lower sensitivity and throughput, thus making them unsuitable for the detection of very low concentrations of the target analyte. Specifically, SPR technology has certain drawbacks. For example, immunoassays using SPR technology can be expensive, may require complex microfluidics systems and high precision optics, may require complex assays, and is a niche technology with few specialists.

SPR technology can be incorporated in, for example, a DMF cartridge. In DMF, droplet operations in the DMF cartridge may occur in a bulk filler fluid (e.g., a low-viscosity oil, such as silicone oil or hexadecane filler fluid). In another example, droplet operations in the DMF cartridge may occur in air, and the droplets may have a thin oil coating (or oil shell) thereon. Further embodiments of this technology may use droplet operations in air without an oil shell.

### SUMMARY

A first aspect includes a cartridge for use with an instrument to perform measurement of a fluid. The cartridge includes a digital microfluidics (DMF) portion comprising a plurality of droplet actuators operative to perform droplet operations on a fluid droplet in the DMF portion and a reaction portion comprising sensor media that is disposed in relation to the plurality of droplet actuators, wherein the plurality of droplet actuators are operative to induce movement of the fluid droplet relative to the sensor media while in contact with the sensor media.

A number of feature refinements and additional features are applicable to the first aspect. These feature refinements and additional features may be used individually or in any combination. As such, each of the following features that will be discussed may be, but are not required to be, used with any other feature or combination of features of the first aspect.

For instance, in an example, the plurality of droplet actuators may comprise reaction electrodes. The plurality of reaction electrodes may perform droplet operations by electrowetting.

In an example, the sensor media of the cartridge may comprise surface plasmon resonance (SPR) sensor media. The SPR sensor media may be functionalized with a capture molecule to which a target molecule of an analyte fluid binds to change an optical signal of the SPR sensor media. The capture molecule may include a ligand immobilized on the SPR sensor medium that is sensitive to binding with the target molecule of the analyte fluid to change an optical property of the SPR sensor media resulting in the change of the optical signal of the SPR sensor media. The change of the optical properties may be a change in the optical signal resulting from light interacting with the SPR sensor media.

In an example, the cartridge may include an SPR sensor surface disposed in the reaction portion and in relation to the plurality of reaction electrodes. The SPR sensor media may be disposed on the SPR sensor surface, and the droplet may be contactingly engageable with the SPR sensor surface by operation of the plurality of reaction electrodes. The SPR sensor media may include one of nanosized structures distributed on the sensor surface or a continuous film comprising nano-sized features. The reaction portion may include a first substrate and second substrate disposed in spaced-apart relation to define a reaction chamber therebetween. The SPR sensor surface may be disposed at the first substrate, and the plurality of reaction electrodes may be disposed at the second substrate opposite the first substrate. Alternatively, the SPR sensor surface may be disposed at the first substrate, and the plurality of reaction electrodes may be disposed at the first substrate.

In an example, the SPR sensor surface may be disposed adjacent to a terminal portion of an optical member comprising at least one optical fiber. The optical member may extend away from one of the first or second substrate to dispose the SPR sensor surface within the reaction chamber. In this example, the optical member may include a first optical fiber on which the optical signal is transmitted from the SPR sensor surface. The optical member may include a second optical fiber on which light from an illumination source is provided to the SPR sensor surface. The optical member may be moveable relative to the first substrate to dispose the SPR sensor surface between an extended position in which the SPR sensor surface is disposed in the reaction chamber and a retracted position in which the SPR sensor surface is not disposed in the reaction chamber. In an example, the reaction chamber contains a filler media. The optical member may be retractable to reduce contact between the SPR sensor surface and the filler media.

In an example, the SPR sensor surface may be disposed between a first reaction electrode and a second reaction electrode. The first reaction electrode and the second reaction electrode may be alternately activated to induce oscillation of the droplet between the first reaction electrode and the second reaction electrode to induce the movement of the fluid droplet relative to the SPR sensor surface. The oscillation of the droplet between the first reaction electrode and the second reaction electrode may be linear. Alternatively, the SPR sensor surface may be disposed between three or more reaction electrodes. The three or more reaction electrodes may be alternately activated to induce oscillation of the droplet between the three or more reaction electrodes to induce the movement of the fluid droplet relative to the SPR sensor surface. In turn, the oscillation of the droplet between the first reaction electrode and the second reaction electrode may be circular.

In an example, the sensor media may include a plurality of sensor nanoparticles suspended in a sensor droplet disposed in the reaction portion. The fluid droplet may be merged with the sensor droplet to form a reacted droplet for measurement of the optical signal of the SPR sensor media in the reacted droplet. The movement induced by the plurality of droplet actuators may be operative to mix the reacted droplet.

In an example, each of the plurality of sensor nanoparticles is magnetically responsive. For instance, each of the plurality of sensor nanoparticles may include a magnetically responsive core. Alternatively, each of the plurality of sensor nanoparticles may include a magnetically responsive element tethered to the sensor nanoparticle. The magnetically responsive element may be physically or chemically coupled to the sensor nanoparticle.

In turn, the cartridge may also include a magnet that is selectively operable to act on the magnetically responsive sensor nanoparticles to immobilize the sensor nanoparticles in the reaction portion to dispose the plurality of nanoparticles in a restrained position relative to the magnet. The plurality of droplet actuators may be operative to move fluid away from the sensor nanoparticles when the magnetically responsive sensor nanoparticles are immobilized by the magnet in the restrained position and to move fluid into contact with the sensor nanoparticles when the magnetically responsive sensor nanoparticles are immobilized by the magnet in the restrained position.

In an example, the movement of the fluid droplet is at a rate greater than a sampling rate of an optical system measuring an optical signal of the sensor media.

In an example, the cartridge may include a plurality of droplet operation electrodes in the DMF portion that are operative to supply fluid to the plurality of droplet actuators. The cartridge may include a reservoir electrode in the DMF portion to receive and maintain the fluid in the DMF portion. The droplet operation may include at least one of droplet merging, droplet splitting, droplet dispensing, or droplet diluting.

In an example, the fluid droplet may include an analyte fluid droplet, and the movement of the analyte fluid droplet relative to the sensor media while in contact with the sensor media may be an effective diffusion rate of the analyte fluid droplet relative to the sensor media. The effective diffusion rate of the analyte fluid droplet may be higher than a binding rate of an analyte relative to the SPR sensor.

In an example, the cartridge includes an electrical contact in electrical communication with the plurality of droplet actuators. The electrical contact may be configured for interface with a controller for control of the plurality of droplet actuators. The cartridge may include a pluggable interface of the cartridge comprising the electrical contact. The pluggable interface may be physically and electrically engageable with an instrument to establish electrical communication between a controller of the instrument and the at least one electrode.

In an example, the reaction portion may be substantially transparent to an illumination source incident on the reaction portion on at least one side of the reaction portion to facilitate real-time optical measurement of the sensor media in a reflectance mode. Alternatively, the reaction portion may be substantially transparent to an illumination source incident on the reaction portion on opposite sides of the reaction portion to facilitate real-time optical measurement of the sensor media in a transmission mode.

In an example the fluid droplet may be an analyte fluid droplet, the SPR sensor media may be operable to detect analyte affinity of the analyte fluid droplet during the movement of the analyte fluid droplet relative to the sensor media, and the analyte affinity may be characterized by an analyte affinity value (K_{D}). The K_{D} may be determined based on an on-rate (K_{ON}) measured during an association phase of the analyte fluid at the SPR sensor and an off-rate (K_{OFF}) measured during a dissociation phase of the analyte fluid at the SPR sensor.

Another aspect of the present disclosure includes a plasmon resonance (PR) system. The PR system includes a cartridge according to the first aspect, including any of the foregoing examples discussed in relation to the first aspect. The system also includes a PR instrument with which the cartridge is engageable. The PR instrument includes a controller in operative communication with the electrical contacts for control of the plurality of droplet actuators and an optical detection system operative to measure an optical signal of the sensor media.

A number of feature refinements and additional features are applicable to the second aspect. These feature refinements and additional features may be used individually or in any combination. As such, each of the following features that will be discussed may be, but are not required to be, used with any other feature or combination of features of the second aspect.

In an example, the optical detection system further comprises an illumination source operative to direct light incident to the sensor media and an optical measurement device that measures the optical signal of the sensor media.

In the PR system of the second aspect, the fluid droplet may be an analyte fluid droplet, and the controller is operative to detect a target molecule in the analyte fluid droplet based on the optical signal of the sensor media in the presence of the analyte fluid droplet while in motion relative to the sensor media. The controller may be operative to measure binding events of the target molecule in the analyte fluid droplet in real time based on the optical signal of the sensor media in while the analyte fluid droplet is in motion relative to the sensor media.

Accordingly, the controller may be operative to determine a quantitative measurement of analyte affinity comprising an analyte affinity value (K_{D}). The K_{D} may be determined based on an on-rate (K_{ON}) measured during an association phase of the sensor media and an off-rate (K_{OFF}) measured during a dissociation phase of the sensor media. The fluid droplet in the reaction portion may be an analyte fluid droplet during the association phase, and the fluid in the reaction portion may be a buffer solution fluid (e.g., a buffer solution fluid droplet) during the dissociation phase.

A third aspect includes a method of operation of a cartridge for measurement of an analyte fluid. The method includes contacting sensor media in a reaction portion of the cartridge with an analyte fluid droplet. The method also includes inducing movement of the analyte fluid droplet with respect to the sensor media while maintaining the analyte fluid droplet in contact with the sensor media. The inducing includes operation of a plurality of droplet actuators disposed relative to the sensor media. The method also includes generating a first optical signal at the sensor media during the movement of the analyte fluid droplet relative to the sensor media.

A number of feature refinements and additional features are applicable to the third aspect. These feature refinements and additional features may be used individually or in any combination. As such, each of the following features that will be discussed may be, but are not required to be, used with any other feature or combination of features of the third aspect.

For instance, in an example, the first optical signal may be an association signal corresponding to an association phase of the sensor media in the presence of the analyte fluid droplet. The method may also include determining an on-rate (K_{ON}) of the analyte fluid droplet based on the association signal. The determining the K_{ON} may include fitting an association curve to the association signal.

In an example, the method also includes moving the analyte fluid droplet from the reaction portion such that the analyte fluid droplet is no longer in contacting engagement with the sensor media. In turn, the method may include introducing a buffer solution fluid droplet to the reaction portion. The buffer solution may be in contacting engagement with the sensor media. The method may also include inducing movement of the buffer fluid droplet with respect to the sensor media while maintaining the buffer fluid droplet in contact with the sensor media. The inducing may include the operation of the plurality of droplet actuators disposed relative to the sensor media. The method may also include generating a second optical signal at the sensor media during the movement of the buffer fluid droplet relative to the sensor media. The second optical signal may be a dissociation signal corresponding to a dissociation phase of the sensor media in the presence of the buffer fluid droplet. In turn, the method may include determining an off-rate (K_{OFF}) of the analyte fluid based on the dissociation signal. The determining the K_{OFF} may include fitting a dissociation curve to the dissociation signal. In an example, the method of the third aspect may include calculating an analyte affinity value (K_{D}) based on the K_{ON} and the K_{OFF}. K_{D} may be the quotient of K_{ON} and K_{OFF}.

In another example, the sensor media may be a plurality of sensor nanoparticles disposed in the reaction portion. The plurality of sensor nanoparticles may be disposed in a sensor droplet. In this regard, the method may also include merging the analyte fluid droplet and the sensor to form a reacted droplet for measurement of the optical signal of the sensor media in the reacted droplet.

In an example, each of the plurality of sensor nanoparticles may be magnetically responsive. In turn, the method may include activating a magnet to dispose the nanoparticles in a restrained position relative to the magnet to immobilize the sensor nanoparticles in the reaction portion. The nanoparticles may be maintained in the restrained position relative to the magnet during moving of a droplet relative to the reaction portion.

In an example, the sensor media may be disposed adjacent to a terminal portion of a moveable member. The method may include moving the moveable member relative to a reaction chamber defined in the reaction portion between an extended position and a retracted position. The sensor media may be disposed in the reaction chamber in the extended position and is removed from the reaction chamber in the retreated position. The reaction chamber may include a filler media. In turn, the method may include retracting the moveable member to the retracted position, introducing a fluid droplet to the reaction portion after the retracting to displace the filler media from an area adjacent to the plurality of droplet actuators, and advancing the moveable member after the introducing to the extended position to dispose the sensor media in the fluid droplet.

In an example, the method includes engaging the cartridge with an instrument. The method may also include measuring a signal from the sensor media while the fluid droplet is moved relative to the sensor media while maintaining the fluid droplet in contact with the sensor media. The sensor media may be SPR sensor media, and the signal may be an optical signal of the SPR sensor media. In turn, the method may include providing light from a light source of the instrument incident to the SPR sensor media. The measuring may include measuring the optical signal of the SPR sensor media at an optical measurement device of the instrument.

In an example, the method may also include establishing electrical communication between a controller of the instrument and the plurality of droplet actuators of the DMF portion and controlling the plurality of droplet actuators of the DMF portion. The inducing movement of the fluid may be in response to the controlling of the plurality of droplet actuators of the DMF portion. In an example, in a first period, the fluid may be a buffer fluid droplet, and the measuring comprises recording a baseline optical signal as the buffer fluid is moved relative to the sensor media while maintaining contact with the sensor media. The method may also include introducing an analyte fluid droplet to the reaction portion in a second period. The measuring may include capturing an association signal corresponding to an association phase of the analyte fluid droplet in the second period. An effective diffusion rate of the analyte fluid droplet relative to the sensor media may be higher than a binding rate of the analyte fluid droplet relative to the sensor media. The method may include determining an on-rate (K_{ON}) of the analyte fluid droplet based on the association signal. The determining the K_{ON} may include fitting an association curve to the association signal.

In a further example, the method may include moving the analyte fluid droplet away from the sensor media and introducing a buffer fluid droplet to the reaction portion in a third period, wherein the measuring comprises capturing a dissociation signal corresponding to a dissociation phase of the analyte in the third period. The method may further include determining an off-rate (K_{OFF}) of the analyte fluid based on the dissociation signal. The determining the K_{OFF} may include fitting a dissociation curve to the dissociation signal. The method may include calculating an analyte affinity value (K_{D}) based on the K_{ON} and the K_{OFF}. K_{D} may be the quotient of K_{ON} and K_{OFF}.

The method may also include supplying, in a fourth period, a regeneration buffer solution fluid droplet to the reaction portion and contacting the regeneration buffer solution fluid droplet with the sensor media to regenerate the sensor media. The method may include functionalizing the sensor media by contacting a functionalization fluid droplet comprising ligands to bind the ligands to the sensor media. The method may also include activating the sensor media by contacting an activation fluid droplet with the sensor media prior to the functionalizing of the sensor media.

This Summary is provided to introduce a selection of concepts in a simplified form that are further described below in the Detailed Description. This Summary is not intended to identify key features or essential features of the claimed subject matter, nor is it intended to be used to limit the scope of the claimed subject matter.

Other implementations are also described and recited herein.

### BRIEF DESCRIPTION OF DRAWINGS

Having thus described the presently disclosed subject matter in general terms, reference will now be made to the accompanying drawings, which are not necessarily drawn to scale, and wherein:
FIG. 1 illustrates a block diagram of an embodiment of the presently disclosed PR system that includes a DMF cartridge that includes LSPR sensing mechanisms for analysis of analytes;
FIG. 2 illustrates a perspective view of one example of the embodiment of the DMF cartridge of the PR system shown in FIG. 1;
FIG. 3 and FIG. 4 show schematic diagrams of an embodiment of an LSPR sensor of the presently disclosed DMF cartridge for analysis of analytes;
FIG. 5A, FIG. 5B, and FIG. 5C illustrate cross-sectional views of a portion of the embodiment of the DMF cartridge shown in FIG. 2 and showing examples of different configurations of the LSPR sensor in relation to droplet operations electrodes;
FIG. 6 and FIG. 7 illustrate plan views of an example of a single LSPR sensor in relation to multiple droplet operations electrodes, which is one example of an embodiment of fixed LSPR sensing of the presently disclosed PR system and DMF cartridge;
FIG. 8 and FIG. 9 illustrate plan views of an embodiment of multiple LSPR sensors in relation to multiple droplet operations electrodes, which is another example of fixed LSPR sensing of the presently disclosed PR system and DMF cartridge;
FIG. 10A and FIG. 10B illustrate side views of an embodiment using a fiber optic probe and an LSPR sensor for fixed LSPR sensing in the DMF cartridge;
FIG. 11A and FIG. 11B illustrate side views of another embodiment of using a fiber optic probe and an LSPR sensor for fixed LSPR sensing in the DMF cartridge;
FIG. 12A and FIG. 12B illustrate side views of yet another embodiment of using a fiber optic probe and an LSPR sensor for fixed LSPR sensing in the DMF cartridge;
FIG. 13A illustrates a side view of an example of a fiber optic probe and an LSPR sensor provided through the bottom substrate of the DMF cartridge;
FIG. 13B illustrates a side view of an example of a fiber optic probe and an LSPR sensor provided through the side of the DMF cartridge and between the bottom and top substrates;
FIG. 13C and FIG. 13D illustrates side views of an example of using robotics to move a fiber optic probe and an LSPR sensor in and out of the DMF cartridge;
FIG. 14A, FIG. 14B, FIG. 14C, FIG. 15, and FIG. 16 illustrate examples of various arrangements of multiple LSPR sensors for processing multiple droplets;
FIG. 17 illustrates a flow diagram of an example of a method of using the DMF cartridge, fixed LSPR sensing processes, and droplet operations for analysis of analytes;
FIG. 18 illustrates a block diagram of an example of an optical system of the presently disclosed PR system that includes a DMF cartridge and LSPR sensing mechanisms for analysis of analytes;
FIG. 19 and FIG. 20 illustrate side views of a portion of the DMF cartridge and an embodiment of a process of in-solution LSPR sensing;
FIG. 21A and FIG. 21B illustrate side views of an embodiment of magnetically responsive nanoparticles that may be used in the in-solution LSPR sensing of the presently disclosed PR system and DMF cartridge;
FIG. 22 illustrates a flow diagram of an embodiment of an in-solution process of functionalizing magnetically responsive nanoparticles using droplet operations;
FIG. 23A through FIG. 23F illustrate pictorially certain steps of the in-solution process shown in FIG. 22,
FIG. 24A, FIG. 24B, FIG. 24C, and FIG. 24D illustrate an embodiment of an in-solution LSPR sensing process in the DMF cartridge wherein droplet operations occur in air;
FIG. 25A, FIG. 25B, FIG. 25C, and FIG. 25D illustrate another embodiment of an in-solution LSPR sensing process in the DMF cartridge wherein droplet operations occur in air with oil-covered droplets;
FIG. 26A, FIG. 26B, FIG. 26C, and FIG. 26D illustrate yet another embodiment of an in-solution LSPR sensing process in the DMF cartridge wherein droplet operations occur in oil (or filler fluid);
FIG. 27 illustrates a flow diagram of an embodiment of a method of using the DMF cartridge, in-solution LSPR sensing processes, and droplet operations for analysis of analytes;
FIG. 28 illustrates a side view of an embodiment of the optical detection system operating in transmission mode in relation to fixed LSPR sensing in the DMF cartridge;
FIG. 29 illustrates a side view of an embodiment of the optical detection system operating in reflection mode in relation to fixed LSPR sensing in the DMF cartridge;
FIG. 30 illustrates a side view of an embodiment of the optical detection system operating in transmission mode in relation to in-solution LSPR sensing in the DMF cartridge;
FIG. 31 illustrates a side view of an embodiment of the optical detection system operating in reflection mode in relation to in-solution LSPR sensing in the DMF cartridge;
FIG. 32 illustrates a side view of an embodiment of the DMF cartridge that includes an optical aperture for use with the optical detection system;
FIG. 33 shows an example of a plot of an SPR response vs. time for first a drop of 1% glycerol in DI water followed by a drop of 2% glycerol in DI water;
FIG. 34 shows an example of a plot of the peak position for activating a carboxyl-gold optical fiber, binding Protein A, blocking with ethanolamine, and binding IgG;
FIG. 35 shows an example of a plot of the protein A/IgG experimental data from both the DMF instrument as well as the OpenSPR and showing an improvement in sensitivity and reduction in dispersion; and
FIG. 36 shows an example of a plot that shows the fitting of binding kinetics to the DMF Protein A data.

### DETAILED DESCRIPTION

The presently disclosed subject matter now will be described more fully hereinafter with reference to the accompanying drawings, in which some, but not all embodiments of the presently disclosed subject matter are shown. Like numbers refer to like elements throughout. The presently disclosed subject matter can be embodied in many different forms and should not be construed as limited to the embodiments set forth herein; instead, these embodiments are provided so that this disclosure will satisfy applicable legal requirements. Indeed, many modifications and other embodiments of the presently disclosed subject matter set forth herein will come to mind to one skilled in the art to which the presently disclosed subject matter pertains having the benefit of the teachings presented in the foregoing descriptions and the associated drawings. Therefore, it is to be understood that the presently disclosed subject matter is not to be limited to the specific embodiments disclosed and that modifications and other embodiments are intended to be included within the scope of the appended claims.

In some embodiments, the presently disclosed subject matter may provide a plasmon resonance (PR) system and instrument, digital microfluidic (DMF) cartridge, and methods of using localized surface plasmon resonance (LSPR) and droplet operations for analysis of analytes.

In one embodiment, the PR system can be an LSPR system, wherein the LSPR system includes a DMF cartridge that further includes LSPR sensing capability for analysis of analytes. The DMF cartridge can be used to facilitate DMF capabilities generally for merging, splitting, dispensing, diluting, transporting, and other types of droplet operations. One application of these DMF capabilities may be for sample preparation. Further, the DMF cartridge may include LSPR sensing means for (1) detecting, for example, certain molecules (e.g., target analytes) and/or chemicals in the sample, and (2) analysis of analytes, such as for measuring binding events in real time to extract ON-rate information, OFF-rate information, affinity information, avidity, aggregation, specificity information, conformation changes, thermodynamic parameters, and/or other data/information related to the molecules under study. Another application of this embodiment may be to explore the interactions between drugs and biomolecules or to study the chemical properties of polymers.

In some embodiments, the LSPR sensing capability of the DMF cartridge may include fixed LSPR sensing operations that can be performed using droplet operations. In other embodiments, the LSPR sensing capability of the DMF cartridge may include in-solution LSPR sensing, which is LSPR sensing processes that can occur in the droplets themselves and using droplet operations. In some embodiments, the LSPR sensing capability of the DMF cartridge may include various arrangements of multiple LSPR sensors for processing multiple droplets.

In some embodiments of the presently disclosed PR system, PR instrument, DMF cartridge, and method, the diffusion or flow rate of the fluid at the LSPR sensor may be faster than the binding rate, thereby increasing the likelihood that the LSPR sensing is measuring the binding rate and is not limited by a slow diffusion or flow rate. In the DMF cartridge, droplet operations may be used to, for example, oscillate a droplet back and forth or in circular motion to generate flow at the fixed LSPR sensing processes and/or to generate mixing in the in-solution LSPR sensing processes and thereby increasing the flux of the molecules to the surface of the LSPR sensor. In other embodiments, the LSPR sensing processes may include vibrating the LSPR sensor itself with respect to, for example, a droplet to generate flow and/or to generate mixing. Accordingly, in the presently disclosed PR system, PR instrument, DMF cartridge, and method, continuous uni-directional flow of the sample at the LSPR sensor may not be required. Instead, a sample or analyte droplet may be manipulated rapidly at the LSPR sensing elements using droplet operations to generate a recirculating flow.

A PR instrument of the PR system may include the DMF cartridge, an optical detection system, and a controller. The optical detection system may include, for example, an illumination source and an optical measurement device in relation to the LSPR sensing elements. In some embodiments, the optical detection system may operate in transmission mode while in other embodiments, the optical detection system may operate in reflection mode. The controller may be provided for controlling the droplet manipulation by activating/deactivating electrodes in the DMF cartridge. The controller also may manage the overall operations of the PR system. Additionally, methods of using droplet operations in the DMF cartridge to perform fixed LSPR sensing operations and/or in-solution LSPR sensing operations may be provided.

In some embodiments of the presently disclosed PR system, PR instrument, DMF cartridge, and method, when bulk filler fluid (e.g., oil, such as silicone oil or hexadecane filler fluid) is present and/or when oil-covered droplets are present, the fixed LSPR sensing and/or the in-solution LSPR sensing may be designed to minimize oil contamination at the LSPR sensing elements.

In some embodiments, the presently disclosed PR system, PR instrument, DMF cartridge, and method can be used to determine the K_{D} value, the K_{ON} value, and/or the K_{OFF} value of the analyte sample with an immobilized ligand, wherein the K_{D} value is a quantitative measurement of affinity between the analyte and ligand, the K_{ON} value indicates the kinetic ON-rate of binding, and the K_{OFF} value indicates the kinetic OFF-rate of binding.

In some embodiments, the fixed LSPR sensors may include a surface with nanostructures immobilized thereon and wherein the nanostructures can be functionalized with capture molecules, such as ligands. Then, a sample droplet that has the target analyte suspended therein may come into contact with the fixed LSPR sensor and wherein the target analyte can be a binding partner with the capture molecules of the fixed LSPR sensor. Then, the optical detection system may be used to capture the real-time kinetic measurements (e.g., the association phase (i.e., K_{ON} value), the dissociation phase (i.e., K_{OFF} value), and the analyte affinity (i.e., K_{D} value)) of the binding process.

In some embodiments, the in-solution LSPR sensing processes may include (1) an LSPR droplet that has nanostructures suspended therein and wherein the nanostructures are functionalized with capture molecules, such as ligands, (2) a sample droplet that has the target analyte suspended therein and wherein the target analyte can be a binding partner with the capture molecules in the LSPR droplet, (3) a process of merging (and/or mixing) the LSPR droplet with the sample droplet using droplet operations such that binding can occur between the capture molecules (e.g., ligands) and the target analyte in the merged droplet, and (4) using the optical detection system, a process of capturing the real-time kinetic measurements (e.g., the association phase (i.e., K_{ON} value), the dissociation phase (i.e., K_{OFF} value), and the analyte affinity (i.e., K_{D} value)) of the binding process.

In some embodiments, the nanostructures suspended in the LSPR droplet of the in-solution LSPR sensing processes may be magnetically responsive nanostructures.

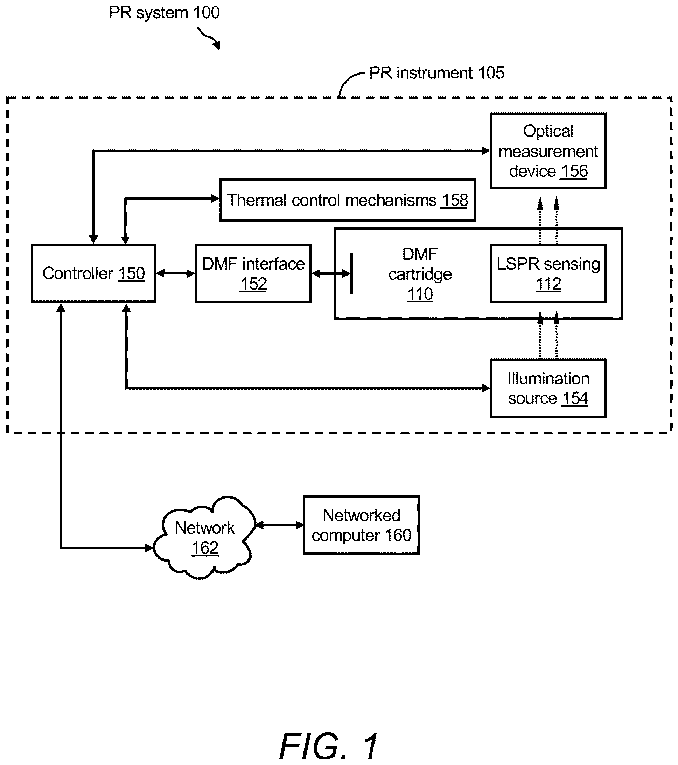

FIG. 1 illustrates a block diagram of an embodiment of the presently disclosed PR system 100 that includes a DMF cartridge that may include LSPR sensing mechanisms for analysis of analytes. Accordingly, PR system 100 can be an LSPR system, wherein PR system 100 includes a DMF cartridge 110 that further includes LSPR sensing capability for analysis of analytes. In PR system 100 for analysis of analytes, analysis can mean, for example, detection, identification, quantification, or measuring analytes and/or the interactions of analytes with other substances, such as binding kinetics. Exemplary analytes may include, but are not limited to, small molecules, proteins, peptides, antibodies, nucleic acids, atoms, ions, polymers, and the like. For example, PR system 100 can be used to measure the binding kinetics of a ligand to a macromolecule, such as a receptor.

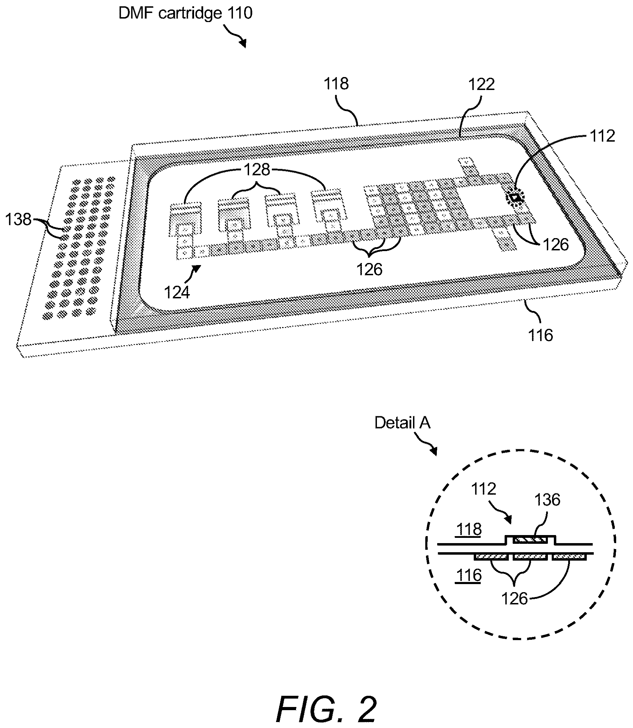

DMF cartridge 110 may facilitate DMF capabilities generally for fluidic actuation including droplet merging, splitting, dispensing, diluting, and the like. One application of these DMF capabilities may be sample preparation. However, the DMF capabilities may be used for other processes, such as waste removal. DMF cartridge 110 of PR system 100 can be provided, for example, as a disposable and/or reusable cartridge. More details of an example of DMF cartridge 110 are shown and described hereinbelow with reference to FIG. 2.

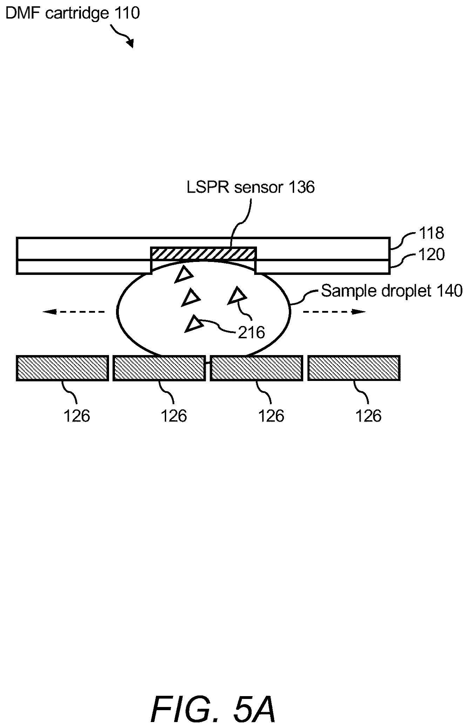

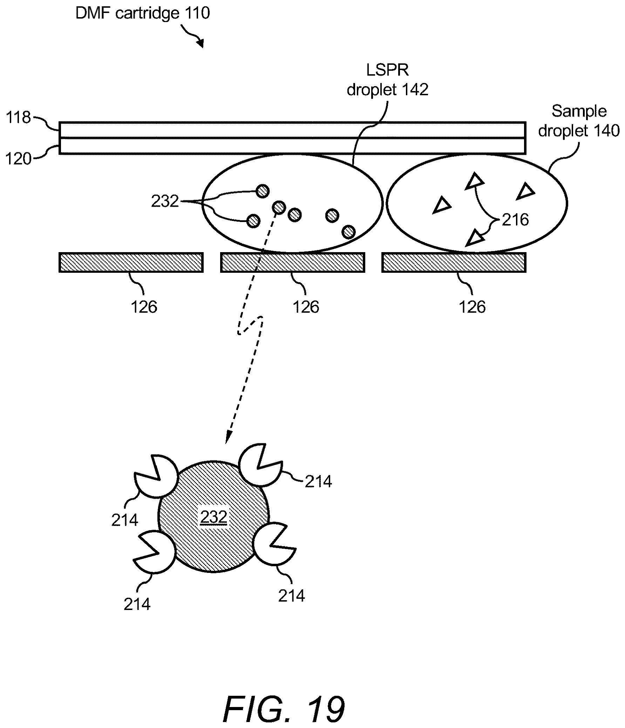

Further, DMF cartridge 110 may include LSPR sensing 112. LSPR sensing 112 may be used for (1) detecting, for example, certain molecules (e.g., target analytes) and/or chemicals in the sample, and/or (2) for analysis of analytes, such as for measuring binding events in real time to extract ON-rate information, OFF-rate information, and/or affinity information. LSPR sensing 112 can be, for example, fixed LSPR sensing and/or any in-solution LSPR sensing processes. Additionally, the fixed LSPR sensing and the in-solution LSPR sensing may be designed to minimize oil contamination at the LSPR sensing elements. More details of fixed LSPR sensing are shown and described hereinbelow with reference to FIG. 5A through FIG. 18. More details of in-solution LSPR sensing are shown and described hereinbelow with reference to FIG. 19 through FIG. 27.

While the discussion presented herein may involve use of an SPR sensor (e.g., an LSPR sensor in certain embodiments), it is contemplated that other sensors can also be used in place of or in addition to the SPR or LSPR sensor. Such alternative or additional sensor options may include electronic sensors, electrochemical sensors, mechanical sensors, or other appropriate sensor types. For example, sensors may be used, such as biolayer interferometry or piezoelectric sensors. In this regard, the interaction between an analyte and a sensor using the DMF capabilities described herein for sample/sensor interaction may generally be applicable for analysis of an analyte using any appropriate sensor.

PR system 100 may further include a controller 150, a DMF interface 152, an illumination source 154, an optical measurement device 156, and thermal control mechanisms 158. Controller 150 may be electrically coupled to the various hardware components of PR system 100, such as to DMF cartridge 110, illumination source 154, and an optical measurement device 156. In particular, controller 150 may be electrically coupled to DMF cartridge 110 via DMF interface 152, wherein DMF interface 152 may be, for example, a pluggable interface for connecting mechanically and electrically to DMF cartridge 110. Together, DMF cartridge 110, controller 150, DMF interface 152, illumination source 154, and optical measurement device 156 may comprise a PR instrument 105.

Controller 150 may, for example, be a general-purpose computer, special purpose computer, personal computer, microprocessor, or other programmable data processing apparatus. Controller 150 may provide processing capabilities, such as storing, interpreting, and/or executing software instructions, as well as controlling the overall operations of PR system 100. The software instructions may comprise machine readable code stored in non-transitory memory that is accessible by the controller 150 for the execution of the instructions. Controller 150 may be configured and programmed to control data and/or power aspects of these devices. For example, with respect to DMF cartridge 110, controller 150 may control droplet manipulation by activating/deactivating electrodes. Generally, controller 150 can be used for any functions of the PR system 100. For example, controller 150 can be used to authenticate the DMF cartridge 110 in a fashion similar to how printer manufacturers check for their branded ink cartridges, controller 150 can be used to verify that the DMF cartridge 110 is not expired, controller 150 can be used to confirm the cleanliness of the DMF cartridge 110 by running a certain protocol for that purpose, and so on.

Additionally, in some embodiments, DMF cartridge 110 may include capacitive feedback sensing. For example, a signal may be generated or detected by a capacitive sensor that can detect droplet position, velocity, and size. Further, in other embodiments, instead of capacitive feedback sensing, DMF cartridge 110 may include a camera or other optical device to provide an optical measurement of the droplet position, velocity, and size, which can trigger controller 150 to re-route the droplets at appropriate positions. The feedback can be used to create a closed-loop control system to optimize droplet actuation rate and verify droplet operations are completed successfully.

Optionally, PR instrument 105 can be connected to a network. For example, controller 150 may be in communication with a networked computer 160 via a network 162. Networked computer 160 can be, for example, any centralized server or cloud server. Network 162 can be, for example, a local area network (LAN) or wide area network (WAN) for connecting to the internet.

In PR system 100, illumination source 154 and optical measurement device 156 may be arranged with respect to LSPR sensing 112 (e.g., fixed LSPR sensing and/or in-solution LSPR sensing) of DMF cartridge 110. The illumination source 154 may be, for example, a light source for the visible range (400-800 nm), such as, but not limited to, a white light-emitting diode (LED), a halogen bulb, an arc lamp, an incandescent lamp, lasers, and the like. Illumination source 154 is not limited to a white light source. Illumination source 154 may be any color light that is useful in PR system 100. Optical measurement device 156 may be used to obtain LSPR light intensity readings. Optical measurement device 156 may be, for example, a charge coupled device, a photodetector, a spectrometer, a photodiode array, or any combinations thereof. Further, PR system 100 is not limited to one illumination source 154 and one optical measurement device 156 only. PR system 100 may include multiple illumination sources 154 and/or multiple optical measurement devices 156 to support multiple LSPR sensing elements. Thermal control mechanisms 158 may be any mechanisms for controlling the operating temperature of DMF cartridge 110. Examples of thermal control mechanisms 158 may include Peltier elements and resistive heaters.

FIG. 2 illustrates a perspective view of one example of DMF cartridge 110 of PR system 100 shown in FIG. 1. DMF cartridge 110 may include a bottom substrate 116 and a top substrate 118. In one example, bottom substrate 116 can be a material that is substantially transparent to white light, such as glass, plastic, or a class of polymers known as thermoplastic elastomers (TPE). In another example, bottom substrate 116 can be a printed circuit board (PCB) that is substantially transparent or one that includes holes or openings that allow light transmission. Further, a set of electrical contacts 138 may be provided on one or both ends of bottom substrate 116. Electrical contacts 138 may be used, for example, to connect to DMF interface 152, and then to controller 150. Like bottom substrate 116, top substrate 118 may be formed of a material that is substantially transparent to white light, such as glass, plastic, or TPE. Further, the inner surface of top substrate 118 may be coated with a transparent conductive layer, such as indium tin oxide (ITO), poly(3,4-ethylenedioxythiophene) polystyrene sulfonate (PEDOT:PSS), or other similar transparent conductive coatings. In other embodiments, all areas of DMF cartridge 110 need not include substantially transparent substrates and/or coatings or layers. For example, the substrates and/or coatings or layers may not be transparent, translucent, and/or opaque except in the region of detection.

The terms "top," "bottom," "over," "under," "in," and "on" are used throughout the description with reference to the relative positions of components of the DMF cartridge, such as relative positions of top and bottom substrates of the DMF cartridge. It will be appreciated that the DMF cartridge is functional regardless of its orientation in space.

In DMF cartridge 110, a gap between the bottom substrate 116 and top substrate 118 may define a reaction (or assay) chamber 122. For example, reaction (or assay) chamber 122 may comprise a space between the bottom substrate 116 and top substrate 118 for processing any liquids of interest via droplet operations; liquids, such as, but not limited to, liquid reagents, buffer solution, sample droplets, and the like. Accordingly, an electrode arrangement 124 may be provided atop bottom substrate 116 in reaction (or assay) chamber 122. Electrode arrangement 124 may include, for example, any arrangement of droplet operations electrodes 126 (e.g., electrowetting electrodes) and reservoir electrodes 128. For example, electrode arrangement 124 may include any lines or paths of droplet operations electrodes 126 in relation to any number of reservoir electrodes 128.

Electrode arrangement 124 may be used for performing droplet operations via electrowetting. "Droplet operation" means any manipulation of a droplet on a DMF device or cartridge. A droplet operation may, for example, include: loading a droplet into the DMF device; dispensing one or more droplets from a source droplet; splitting, separating or dividing a droplet into two or more droplets; transporting a droplet from one location to another in any direction; merging or combining two or more droplets into a single droplet; diluting a droplet; mixing a droplet; agitating a droplet; deforming a droplet; retaining a droplet in position; incubating a droplet; heating a droplet; vaporizing a droplet; cooling a droplet; disposing of a droplet; transporting a droplet out of a droplet actuator; other droplet operations described herein; and/or any combination of the foregoing. Further, for controlling the temperature of processes occurring in reaction (or assay) chamber 122, a temperature control element (not shown), such as a Peltier heat pump, can be used in combination with DMF cartridge 110.

Further, while FIG. 2 describes the DMF cartridge 110 as manipulating droplets via electrowetting methods (e.g., using droplet operations electrodes 126), this is exemplary only. In other embodiments, droplets may be manipulated in DMF cartridge 110 via other methods, such as, but not limited to, optical methods, magnetic methods, thermocapillary methods, surface acoustic wave methods, and the like, and any combinations thereof.

By way of example, an instance of LSPR sensing 112 is shown with respect to a line or path of droplet operations electrodes 126. In one example and referring now to Detail A of FIG. 2, LSPR sensing 112 may include an LSPR sensor 136 integrated into or near the droplet operations gap of DMF cartridge 110 and in relation to droplet operations electrodes 126. LSPR sensor 136 can be used for (1) detecting, for example, certain molecules (e.g., target analytes) and/or chemicals in the sample, and/or (2) for analysis of analytes, such as for measuring binding events in real time to extract ON-rate information, OFF-rate information, and/or affinity information.

As described herein, "localized surface plasmon resonance (LSPR)" means using nanoparticle-based or nanostructure-based transducers to monitor binding events in real time without additional labels. For example, nanoparticle-based transducers may include metal nanoparticles from about 1 nm to about 1000 nm in various dimensions. For example, nanostructure-based transducers may include gold films that include nano-sized features (e.g., nano-sized bumps, posts, holes, ridges, lines, and the like.) Some nanoparticle-based or nanostructure-based diagnostic assays are "label-free."

LSPR is a phenomenon associated with noble metal nanoparticles that creates sharp spectral absorbance and scattering peaks and produces strong electromagnetic near-field enhancements. These spectral peaks may be monitored using absorbance spectroscopy. The spectral peak changes with refractive index changes in the immediate vicinity of the nanoparticle surface. When chemical targets are bound near the surface of a metal nanoparticle, a shift in the spectral peak occurs due to changes in the local refractive index. This may be used to determine the concentration of a specific target in a complex medium. Alternatively, the spectral peak shift may be detected through a change in absorption at a given wavelength.

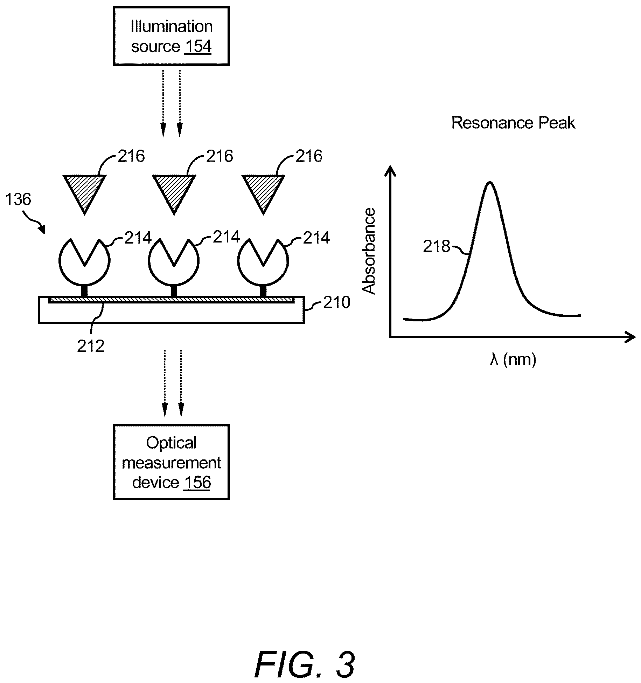

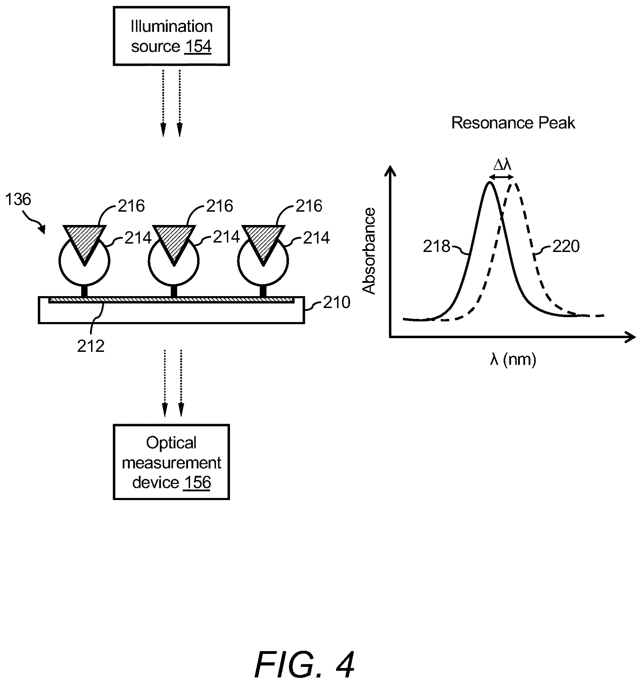

LSPR sensors operate through the immobilization of metal nanoparticles onto a solid support that may include, for example, a flat surface or a microstructured surface. The nanoparticles may be functionalized with specific capture molecules, which may be an antibody. The sample of interest may be flowed over the top of the metal nanoparticles, the target chemicals of interest bind to their respective capture molecules, and the overall spectral peak of the sensor shifts according to the concentration of the chemical target on the capture molecules. LSPR sensors with nanoparticles on planar surfaces operate by flowing the sample longitudinally over the surface. In order to measure this shift, reflectance or transmission absorbance spectroscopy may be employed. Further, analysis via "intensity or colorimetric methods" may be performed using LSPR sensors. More details of examples of LSPR sensors are shown and described hereinbelow with reference to FIG. 3 and FIG. 4.

FIG. 3 and FIG. 4 show schematic diagrams of an embodiment of an LSPR sensor 136 of the presently disclosed DMF cartridge 110 for analysis of analytes. Generally, LSPR may be label-free interaction analysis in real-time. However, LSPR can also be used with labels to enhance the signal. The basic structure of the assay may include a sensor chip (e.g., LSPR sensor 136) that may comprise a glass or plastic substrate with a surface that produces LSPR, such as a collection of discrete nanostructures distributed on a surface, or a continuous film that has nano-sized features formed therein. Then, one of two binding partners may be immobilized on the surface of the sensor. In LSPR, the "ligand" may refer to the binding partner that may be immobilized on the surface of the sensor. The "analyte" may refer to what flows in solution over the ligand on the surface of the sensor. When the analyte binds to the ligand, it changes the optical properties of the surface of the sensor, which is measurable in real time.

In this example, LSPR sensor 136 may include a substantially transparent or opaque substrate 210, such as glass, plastic, or TPE substrate. That is, substrate 210 may be substantially transparent when used in a transmission mode configuration. By contrast, substrate 210 may be opaque when used in a reflection mode configuration. An LSPR sensor layer 212 may be provided atop substrate 210. LSPR sensor layer 212 may be, for example, a gold film that includes certain nanostructures that create an LSPR effect. LSPR sensor layer 212 may be functionalized with one or more capture molecules 214. In one example, capture molecules 214 may comprise ligands that are immobilized on the surface of LSPR sensor layer 212. In this example, the ligands may comprise one of two binding partners, the other binding partner may be a target analyte 216, wherein the target analyte 216 flows in solution over the capture molecules 214 as shown in FIG. 3. By contrast, FIG. 4 shows the target analytes 216 binding to capture molecules 214. This binding may be referred to as a binding event.

Referring now again to FIG. 3, a plot 218 is provided that indicates the optical absorbance peak of LSPR sensor layer 212 prior to a binding event occurring. For example, plot 218 shows the peak position or intensity prior to target analytes 216 binding to capture molecules 214 in LSPR sensor 136. With reference now to FIG. 4, the change in peak position or intensity that is induced by binding of the target analytes 216 to the capture molecules 214 may be monitored in real time. For example, by comparing the peak position prior to binding (i.e., plot 218) with the peak position after binding (i.e., a plot 220). Generally, in LSPR sensor 136, as analytes bind to the surface, the resonance peak of the light will shift to a higher wavelength, which is measurable in real time.

With reference now again to FIG. 1 through FIG. 4, in the presently disclosed PR system 100, PR instrument 105, and DMF cartridge 110, the diffusion or flow rate of the fluid with respect to LSPR sensing 112 (e.g., fixed LSPR sensors 136 and/or in-solution LSPR sensing) may be faster than the binding rate, thereby ensuring that the LSPR sensing 112 is measuring the binding rate and is not limited by a slow diffusion or flow rate. In DMF cartridge 110, droplet operations may be used to, for example, oscillate a droplet back and forth or in circular motion to generate flow at the LSPR sensor and/or to generate mixing in the in-solution LSPR sensing processes and thereby ensure flux of the molecules to the surface of the LSPR sensor. Accordingly, in the presently disclosed PR system 100, PR instrument 105, and DMF cartridge 110, continuous flow of the sample at the LSPR sensor may not be required. Instead, a sample droplet may be manipulated rapidly at the LSPR sensor using droplet operations.

In the presently disclosed PR system 100, PR instrument 105, and DMF cartridge 110, flow can be artificially created by using droplet operations to move the droplet rapidly around on the DMF surface, either back and forth in a line or in a circular method. If the droplet is moved faster than the optical sampling rate, it is likely that artifacts from this movement will have little to no effects on the optical measurements. In another example, if the droplet spans multiple electrodes, the momentum may be changed without moving the droplet off the sensor location and thus avoiding movement artifacts. FIG. 5A through FIG. 26D below show and describe examples of both fixed LSPR sensing and in-solution LSPR sensing of the presently disclosed PR system 100, PR instrument 105, and DMF cartridge 110 that may utilize droplet operations for creating flow rather than using a continuous flow of the sample at the LSPR sensor spot.

### Fixed LSPR sensing in DMF

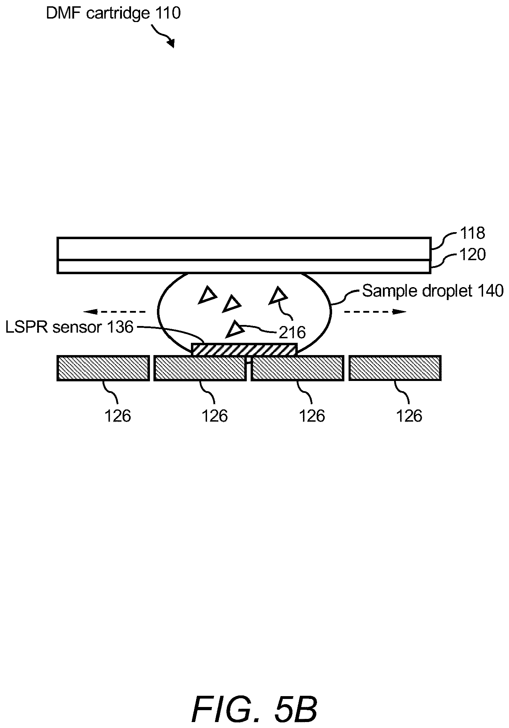

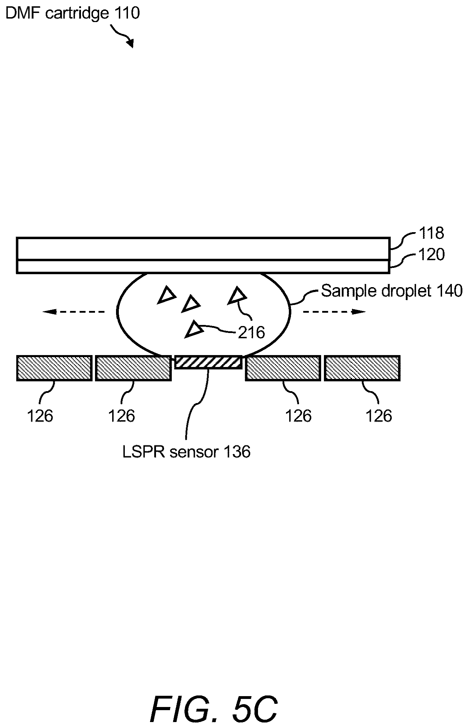

FIG. 5A, FIG. 5B, and FIG. 5C illustrate cross-sectional views of a portion of the embodiment of the DMF cartridge 110 shown in FIG. 2 and showing examples of different configurations of LSPR sensor 136 in relation to droplet operations electrodes 126. Referring now to FIG. 5A is an example of an LPSR detection spot in top substrate 118 of DMF cartridge 110. For example, LSPR sensor 136 may be installed in top substrate 118 of DMF cartridge 110 and opposite any arrangement of droplet operations electrodes 126. A sample (or analyte) droplet 140 may be located in the gap between droplet operations electrodes 126 on bottom substrate 116 and top substrate 118 and wherein sample droplet 140 can be transported along droplet operations electrodes 126 via droplet operations. Sample droplet 140 may include any target analytes 216. When sample droplet 140 is brought into contact with LSPR sensor 136, illumination source 154 and optical measurement device 156 of PR system 100 may be used to capture the real-time kinetic measurements (e.g., the association phase (i.e., K_{ON} value), the dissociation phase (i.e., K_{OFF} value), and the analyte affinity (i.e., K_{D} value)) of the target analytes 216 in sample droplet 140 to the capture molecules 214 (e.g., ligands) of LSPR sensor 136.

Referring now to FIG. 5B is an example of an LPSR detection spot in bottom substrate 116 of DMF cartridge 110 but in a different layer or plane than the droplet operations electrodes 126. For example, LSPR sensor 136 may be installed in bottom substrate 116 and atop certain droplet operations electrodes 126.

Referring now to FIG. 5C is an example of an LPSR detection spot in bottom substrate 116 of DMF cartridge 110 but in the same layer or plane as the droplet operations electrodes 126. For example, LSPR sensor 136 may be installed between and in line with two droplet operations electrodes 126.

In the examples shown in FIG. 5A, FIG. 5B, and FIG. 5C, any LSPR sensor 136 installed in and/or near bottom substrate 116 and/or top substrate 118 of DMF cartridge 110 may be an example of LSPR sensing 112. In particular, any LSPR sensor 136 installed in and/or near bottom substrate 116 and/or top substrate 118 may be an example of fixed LSPR sensing 112 in DMF cartridge 110. Further, in order to help increase the likelihood that the fixed LSPR sensor 136 is measuring the binding rate and is not limited by a slow diffusion or flow rate, LSPR sensor 136 may be used in combination with rapid movement of, for example, sample droplet 140 when at LSPR sensor 136 and wherein this rapid movement is achieved via droplet operations.

In the examples shown in FIG. 5A, FIG. 5B, and FIG. 5C, the position of LSPR sensor 136 may be approximately at the boundary between two droplet operations electrodes 126. Then, using the two droplet operations electrodes 126 and droplet operations, sample droplet 140 may be moved back and forth rapidly between the two droplet operations electrodes 126 and accordingly back and forth rapidly across the surface of LSPR sensor 136. Further, sample droplet 140 may be moved back and forth rapidly at, for example, a rate faster than the sampling rate of the optical measurement system. This may reduce optical artifacts in an obtained signal. In one example, if the optical sampling rate is about 4 Hz (about every 250 ms), then sample droplet 140 may be moved (via droplet operations) back and forth between the two droplet operations electrodes 126 at a rate faster than about 4 Hz.

Further, moving sample droplet 140 (1) helps increase the flux of the molecules to the surface of LSPR sensor 136 and (2) helps improve the likelihood that the binding rate is being measured at LSPR sensor 136 without limitation of diffusion or flow rate. In the DMF cartridge, droplet operations may be used to, for example, oscillate a droplet back and forth or in circular motion to generate flow at the LSPR sensor and/or to generate mixing in the in-solution LSPR sensing processes and thereby help increase flux of the molecules to the surface of the LSPR sensor. In another embodiment, sample droplet 140 can span two or more droplet operations electrodes 126. Then, droplet operations electrodes 126 can be actuated at a rate faster than the bulk droplet can move off of the sensor location, agitating the liquid and similarly promoting flux of the molecules. More details of examples of using fixed LSPR sensing 112 and droplet operations to capture the real-time kinetic measurements of a sample droplet are shown and described hereinbelow with reference to FIG. 5A through FIG. 17.

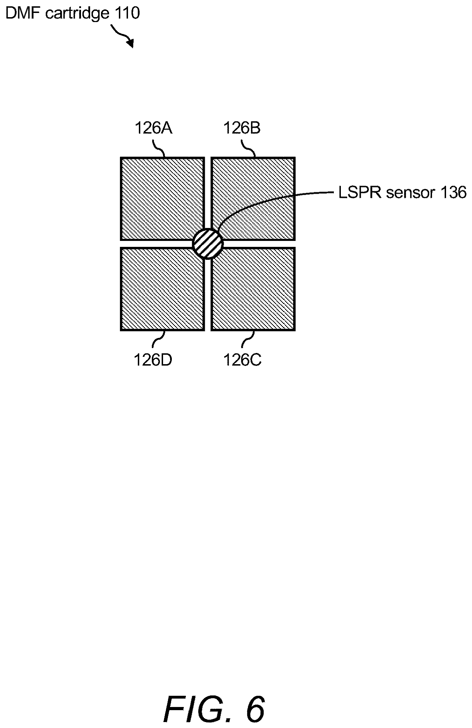

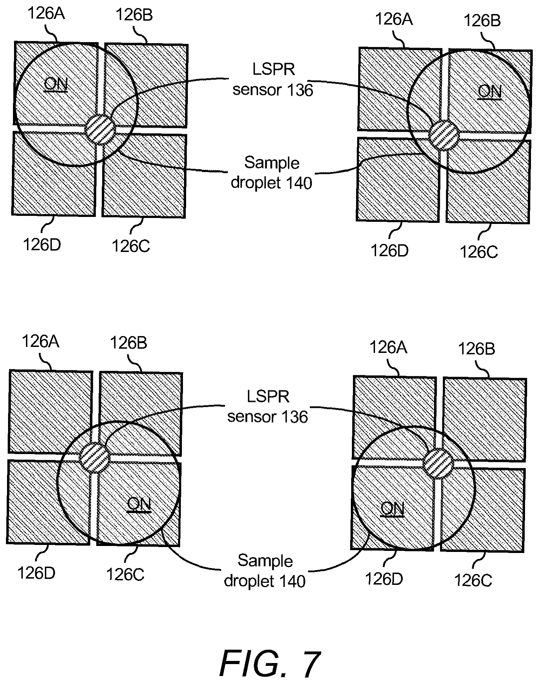

FIG. 6 and FIG. 7 shows plan views of an embodiment of a single LSPR sensor 136 in relation to multiple droplet operations electrodes 126, which is one example of fixed LSPR sensing 112 of the presently disclosed PR system 100 and DMF cartridge 110. Referring now to FIG. 6, the single LSPR sensor 136 may be installed in relation to four droplet operations electrodes 126 that are arranged in 2 x 2 configuration, e.g., droplet operations electrodes 126A, 126B, 126C, 126D. For example, LSPR sensor 136 may be positioned at about the center of the 2 x 2 configuration of droplet operations electrodes 126. Further, the area of LSPR sensor 136 may be relatively small compared with the area of each of the droplet operations electrodes 126 in order to minimize disruption to the droplet movement.

In operation and referring now to FIG. 7, each of the droplet operations electrodes 126 may be activated one at a time. For example, droplet operations electrode 126A may be activated, then droplet operations electrode 126B, then droplet operations electrode 126C, and then droplet operations electrode 126D. Accordingly, sample droplet 140 with the target analytes 216 therein can be moved (using droplet operations) in a circular fashion around the 2 x 2 configuration of droplet operations electrodes 126. In doing so, sample droplet 140 may be moved (using droplet operations) in a circular fashion around the surface of LSPR sensor 136. In this example, the footprint of sample droplet 140 may be about equal to or larger than the footprint (i.e., area) of each of the droplet operations electrodes 126. In this way, sample droplet 140 can be in contact with LSPR sensor 136 regardless on which droplet operations electrode 126 it's positioned on. Again, moving sample droplet 140 back and forth rapidly at, for example, a rate faster than the sampling rate of the optical measurement system (1) may increase flux of the molecules to the surface of LSPR sensor 136 and (2) may increase the likelihood that the binding rate is being measured at LSPR sensor 136 without limitation of slow diffusion or flow rate. Regarding electrode motion, if the droplet is, for example, about 2x the footprint of a unit electrode, then up/down and/or left/right movement is possible by activating both diagonal electrodes at the same time.

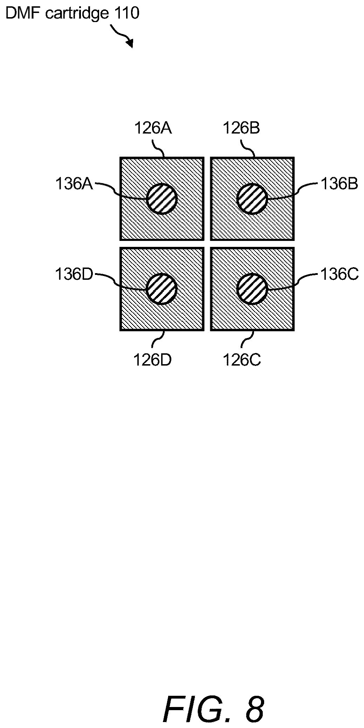

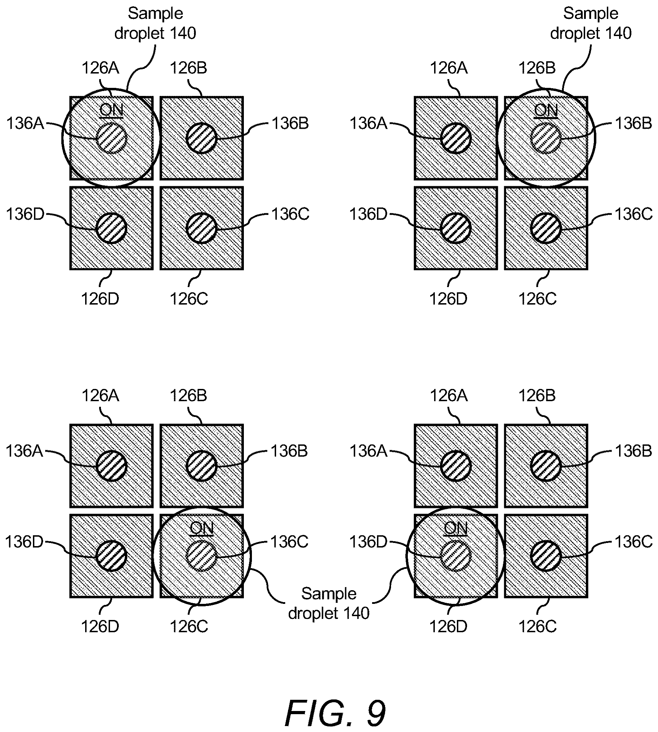

FIG. 8 and FIG. 9 illustrate plan views of an embodiment of multiple LSPR sensors 136 in relation to multiple droplet operations electrodes, which is another example of fixed LSPR sensing 112 of the presently disclosed PR system 100 and DMF cartridge 110. This configuration of multiple fixed LSPR sensors 136 may allow for multiplexed measurements of the sample droplet 140. Referring now to FIG. 8, the 2 x 2 configuration of droplet operations electrodes 126 may be provided, e.g., droplet operations electrodes 126A, 126B, 126C, 126D. Further, each of the droplet operations electrodes 126A, 126B, 126C, 126D may have a corresponding LSPR sensor 136A, 136B, 136C, 136D. For example, an LSPR sensor 136 may be positioned at about the center of each droplet operations electrode 126. Again, the area of each LSPR sensor 136 may be relatively small compared with the area of its corresponding droplet operations electrode 126 in order to minimize disruption to the droplet movement.

In operation and referring now to FIG. 9, each of the droplet operations electrodes 126 may be activated one at a time. For example, droplet operations electrode 126A may be activated, then droplet operations electrode 126B, then droplet operations electrode 126C, and then droplet operations electrode 126D. Accordingly, sample droplet 140 with the target analytes 216 therein can be moved (using droplet operations) in a circular fashion around the 2 x 2 configuration of droplet operations electrodes 126. In so doing, sample droplet 140 may be moved (using droplet operations) in a circular fashion from one LSPR sensor 136 to the next; such as to LSPR sensor 136A, then to LSPR sensor 136B, then to LSPR sensor 136C, and then to LSPR sensor 136D. Again, moving sample droplet 140 back and forth rapidly at, for example, a rate faster than the sampling rate of the optical measurement system (1) may increase flux of the molecules to the surface of LSPR sensors 136 and (2) may increase the likelihood that the binding rate is being measured at LSPR sensors 136 without limitation of slow diffusion or flow rate.

With respect to the droplet patterns described hereinabove with respect to FIG. 6 through FIG. 9, the droplet motion pattern is not limited to a circular motion pattern only. Other motion patterns are possible. For example, the droplet motion pattern can be x-shaped or random. In another example, a certain droplet operations electrode 126 with a droplet thereon can be simply pulsed on and off to agitate the droplet at a certain LSPR sensor 136.

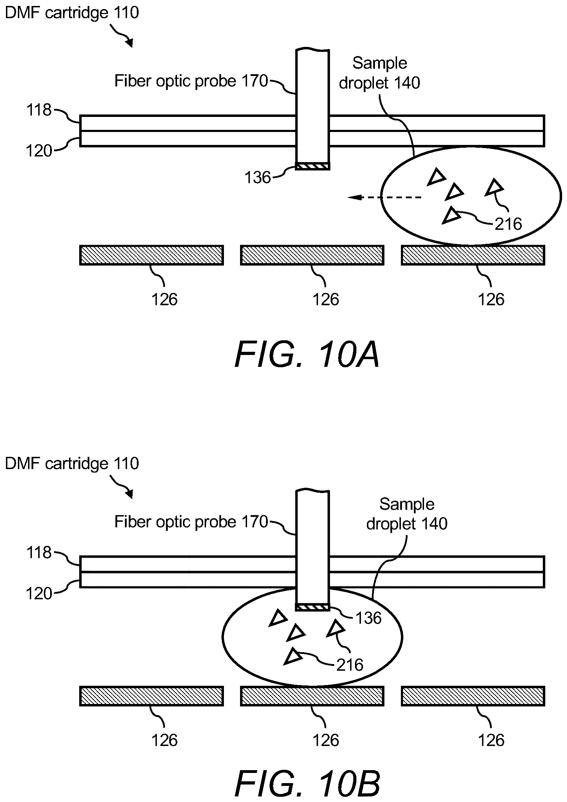

FIG. 10A and FIG. 10B illustrate side views of an embodiment using a fiber optic probe in combination with LSPR sensor 136 for fixed LSPR sensing 112 in DMF cartridge 110. In this example, LSPR sensor 136 may be mechanically and optically coupled to the tip of a fiber optic probe 170. Fiber optic probe 170 can be installed through top substrate 118 such that LSPR sensor 136 extends into the gap between droplet operations electrodes 126 (on bottom substrate 116) and top substrate 118. In one example, when the optical measurement system is operating in transmission mode, fiber optic probe 170 can be coupled to optical measurement device 156 and illumination source 154 may be positioned opposite LSPR sensor 136. In another example, when the optical measurement system is operating in reflection mode, fiber optic probe 170 can actually contain at least two optical fibers wherein at least a first fiber couples to the illumination source 154 and at least another fiber couples to optical measurement device 156. Alternatively, one fiber may be used for both illumination and readout. In other embodiments, LSPR sensor 136 may be built onto this fiber (or fiber bundle).

FIG. 10A and FIG. 10B show an embodiment of a process of transporting sample droplet 140 to fiber optic probe 170 such that fiber optic probe 170 and LSPR sensor 136 may protrude into sample droplet 140. Again, sample droplet 140 can be moved rapidly via droplet operations to increase the flux of the molecules to the surface of LSPR sensor 136. For example, the fiber optic probe 170 and LSPR sensor 136 may be implemented in the configurations described hereinabove with reference to the fixed LSPR sensing 112 shown in FIG. 6 and FIG. 7 and also in FIG. 8 and FIG. 9.

In the process shown in FIG. 10A and FIG. 10B, droplet operations may occur in air. For example, the gap between the bottom substrate 116 and top substrate 118 may be filled with air. However, in other embodiments, droplet operations in DMF cartridge 110 may occur in air, and wherein droplets may have a thin oil coating (or oil shell) thereon. In yet other embodiments, droplet operations in DMF cartridge 110 may occur in a bulk filler fluid (e.g., a low-viscosity oil, such as silicone oil or hexadecane filler fluid). For example, the gap between the bottom substrate 116 and top substrate 118 may be filled with a filler fluid. A drawback of the presence of oil in a DMF cartridge is that there may be a risk of oil contamination at the LSPR sensing elements. However, in PR system 100, a fiber optic probe 170 and LSPR sensor 136 may provide a mechanism to minimize or entirely eliminate oil contamination at the LSPR sensing elements.

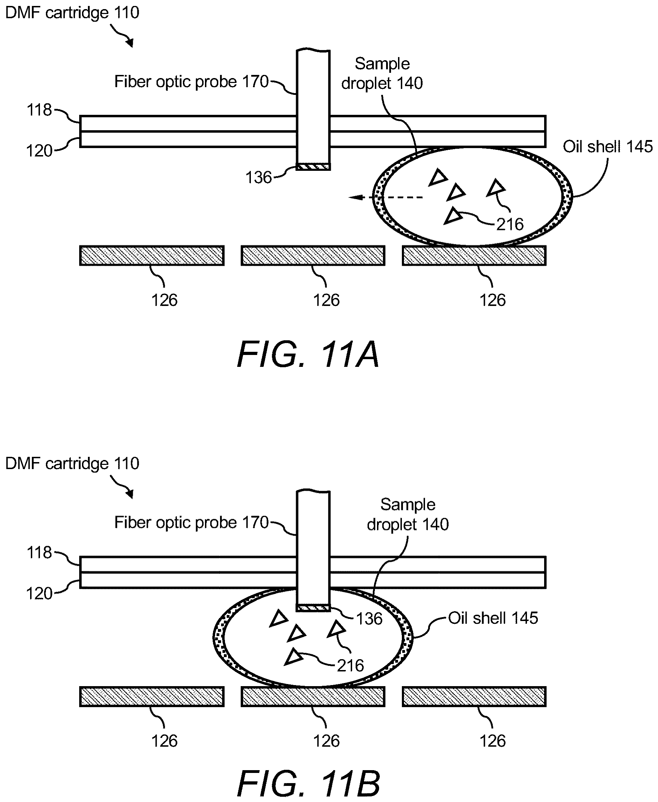

For example and referring now to FIG. 11A and FIG. 11B, sample droplet 140 may have an oil shell (or coating) 145 thereon. When sample droplet 140 is transported to the fiber optic probe 170, the fiber optic probe 170 and LSPR sensor 136 may pass briefly through oil shell 145 and then protrude into the aqueous portion of sample droplet 140. Because the surface of LSPR sensor 136 is generally hydrophilic, there tends to be an aqueous layer on the surface of LSPR sensor 136, which may resist oil contamination and helps promote a reliable reading. Further, this configuration can be used to minimize the amount of time that LSPR sensor 136 is exposed to the oil phase.

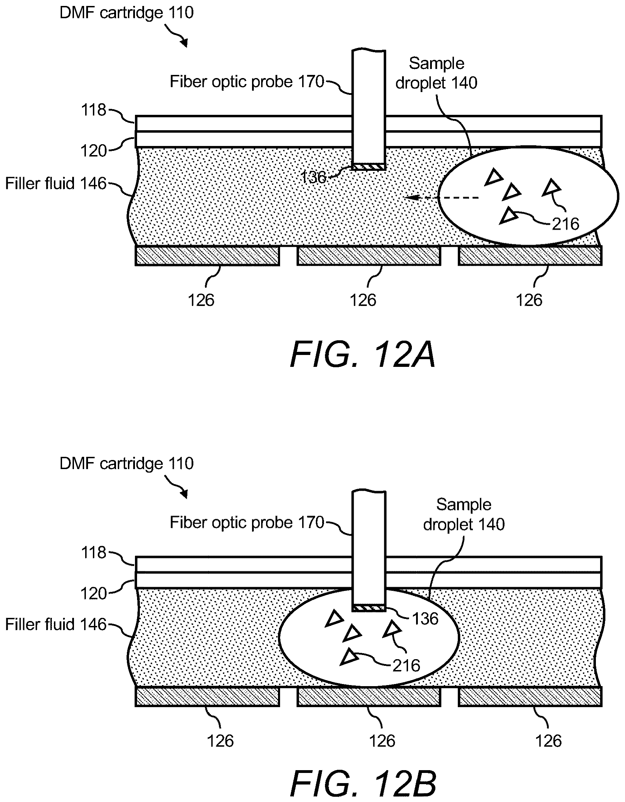

Further to the embodiment and referring now to FIG. 12A and FIG. 12B, the gap between the bottom substrate 116 and top substrate 118 may be filled with filler fluid 146. Filler fluid 146 can be, for example, a low-viscosity oil, such as silicone oil or hexadecane filler fluid. Accordingly, droplet operations in DMF cartridge 110 may occur in the oil. In this example, when sample droplet 140 is transported to the fiber optic probe 170, the fiber optic probe 170 and LSPR sensor 136 transition from the fully oil environment of DMF cartridge 110 to the fully aqueous environment of sample droplet 140. Again, because the surface of LSPR sensor 136 is generally hydrophilic, there tends to be an aqueous layer on the surface of LSPR sensor 136, which resists oil contamination and helps promote a reliable reading.

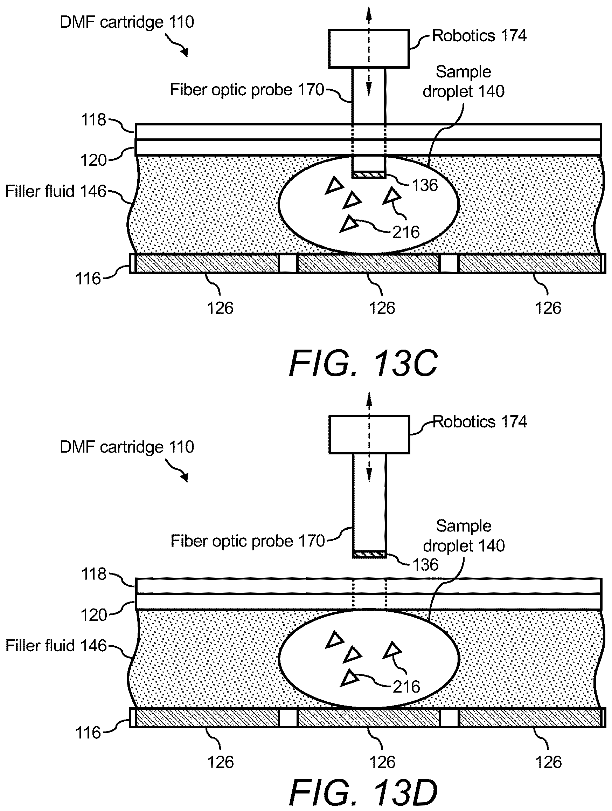

In another embodiment, the fiber optic probe 170 and the DMF cartridge 110 may be disposed for relative movement therebetween. For instance, the fiber optic probe 170 may be selectively displaced from the DMF cartridge 110 to remove the fiber optic probe 170 from between the bottom substrate 116 and the top substrate 118 (see FIG. 13C and FIG. 13D). In this regard, the fiber optic probe 170 may be removed from the DMF cartridge 110 while there is an oil environment in the DMF cartridge 110 between the bottom substrate 116 and the top substrate 118. However, once a droplet is in place, and an aqueous environment is present between the bottom substrate 116 and the top substrate 118, the fiber optic probe 170 may be moved into position such that the LSPR sensor 136 may be disposed in the aqueous environment established by the droplet. In this regard, oil contamination of the LSPR sensor 136 may be minimized by moving the fiber optic probe 170 out of position during droplet operations in which oil may be present in the area occupied by the fiber optic probe 170 when in an inserted position relative to the DMF cartridge 110. However, once a droplet is in position and the area occupied by the fiber optic probe 170 when in an inserted position relative to the DMF cartridge 110 comprises an aqueous environment, the fiber optic probe 170 may be inserted such that the LSPR sensor 136 experiences little to no oil contact. Furthermore, different ones of a plurality of fiber optic probes 170 may be selectively introduced in this manner to allow for the use of new probes and/or probes comprising different kinds of sensors.

With reference now again to FIG. 10A through FIG. 12B, a fiber optic probe 170 and LSPR sensor 136 can be replaceable. For example, once the assay is complete, the fiber optic probe 170 and LSPR sensor 136 can be removed and a new fiber optic probe 170 and LSPR sensor 136 re-inserted through top substrate 118 of DMF cartridge 110.

In the fixed LSPR sensing processes shown in FIG. 5A through FIG. 12B, the analyte solution of sample droplet 140 can be replaced (via droplet operations) with buffer solution as needed to avoid the accumulation of analyte in the sample droplet 140.

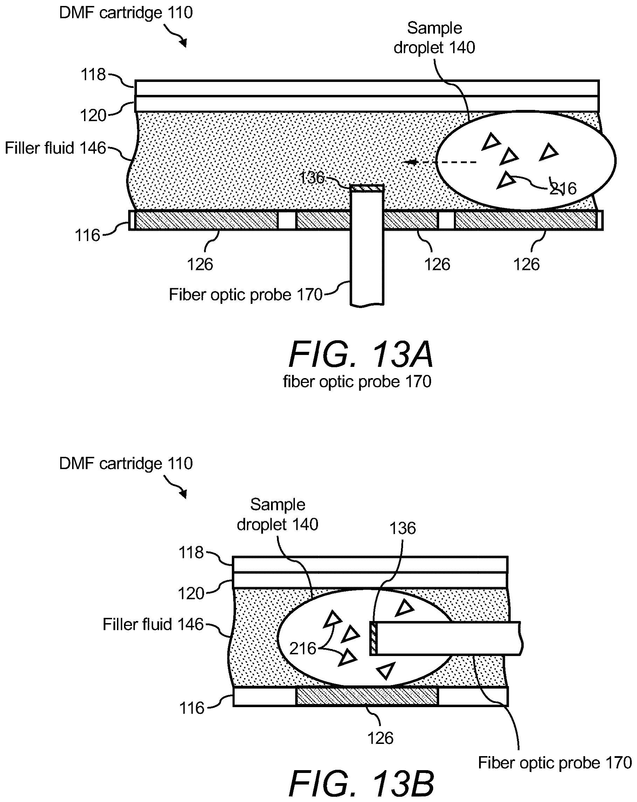

The configuration of the fiber optic probe (e.g., the fiber optic probe 170) with respect to DMF cartridge 110 is not limited to those shown, for example, in FIG. 10A through FIG. 12B. For example, FIG. 13A through FIG. 13D illustrate side views of still other configurations of a fiber optic probe and an LSPR sensor for fixed LSPR sensing in DMF cartridge 110. In one example, FIG. 13A shows fiber optic probe 170 inserted through bottom substrate 116 of DMF cartridge 110 instead of through top substrate 118. In another example, FIG. 13B shows fiber optic probe 170 with LSPR sensor 136 is inserted from the side of DMF cartridge 110 and into the gap between top substrate 118 and bottom substrate 116. In this example, fiber optic probe 170 is oriented parallel to the plane of top substrate 118 rather than perpendicular to the plane of top substrate 118, as shown in FIG. 10A through FIG. 12B. A benefit of this configuration of fiber optic probe 170 is that it may provide minimal perturbation to the fluidic function of the device.

In yet another example, FIG. 13C and FIG. 13D show the use of robotics 174 to move or retract fiber optic probe 170 into or out of the gap between the bottom substrate 116 and top substrate 118 of DMF cartridge 110. In this way, the fiber optic probe 170 is moveable. In another example of moving fiber optic probe 170, vibrating action can be applied to the fiber optic probe 170 to aid in mixing.

In still another example, a robotics system (not shown) may be used to move fiber optic probes 170 with LSPR sensors 136 to specific droplet locations within DMF cartridge 110. For example, the robotics system can be used to move fiber optic probes 170 with LSPR sensors 136 through the bottom substrate 116 and/or top substrate 118 of DMF cartridge 110 and/or into the gap between the bottom substrate 116 and top substrate 118 from the side of DMF cartridge 110. Generally, fiber optic probes 170 can be moved, for example, mechanically, electrically, and/or magnetically within DMF cartridge 110 to different sample lanes and/or locations.

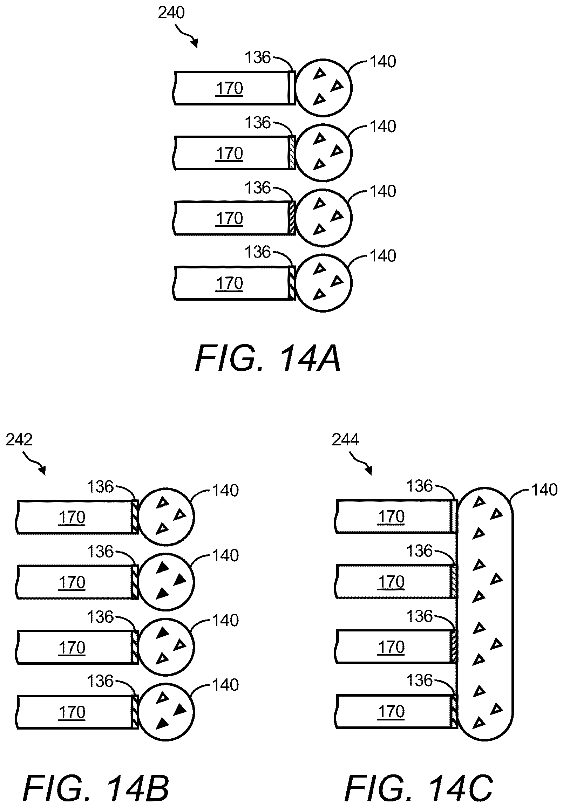

In some embodiments, the LSPR sensing capability of DMF cartridge 110 may include various arrangements of multiple LSPR sensors 136 for processing multiple droplets, examples of which are shown in FIG. 14A, FIG. 14B, FIG. 14C, FIG. 15, and FIG. 16. For example and referring now to FIG. 14A, a sensor configuration 240 is provided that includes, for example, an arrangement of multiple fiber optic probes 170 and LSPR sensors 136, wherein the characteristics (e.g., immobilization chemistry) of the different LSPR sensors 136 may vary while the sample droplets 140 are substantially the same. In another example and referring now to FIG. 14B, a sensor configuration 242 is provided that includes, for example, an arrangement of multiple fiber optic probes 170 and LSPR sensors 136, wherein the characteristics of the different LSPR sensors 136 are substantially the same while the sample droplets 140 are different. In yet another example and referring now to FIG. 14C, a sensor configuration 244 is provided that includes, for example, an arrangement of multiple fiber optic probes 170 and LSPR sensors 136, wherein the characteristics of the different LSPR sensors 136 may vary for processing a single sample droplet 140 that is spanning all of the LSPR sensors 136.

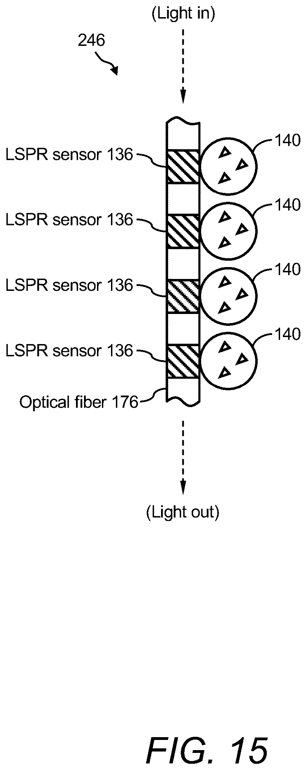

In yet another example and referring now to FIG. 15, a sensor configuration 246 is provided. Sensor configuration 246 includes a single optical fiber 176 that may be arranged, for example, across DMF cartridge 110, wherein optical fiber 176 includes multiple detection spots (e.g., multiple LSPR sensors 136). In one example, the detection spots can be resolved by having different nanostructures on each spot so they are spectrally resolved. In another example, optical fiber 176 with the multiple detection spots (e.g., multiple LSPR sensors 136) may be imaged from above with a camera and then spatially resolved using image processing. In one example, each detection spot (e.g., each LSPR sensor 136) may line up with a row of droplet operations electrodes 126 to be individually addressable. Further, each detection spot (e.g., each LSPR sensor 136) may be on a separate electrode track for individual functionalization/sensing. This method is an example of a way to multiplex a large number of LSPR sensors 136. This method also overcomes certain limits imposed by the physical space available to integrate optical fibers with DMF cartridge 110.

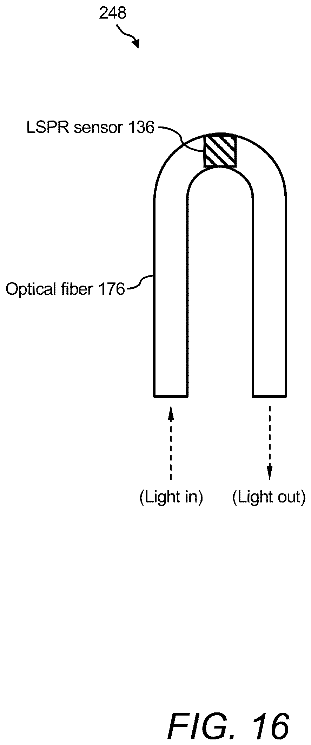

In yet another example and referring now to FIG. 16, a sensor configuration 248 is provided. Sensor configuration 248 includes a U-bent optical fiber 176 that has a detection spot (e.g., LSPR sensor 136) arranged at the U-portion of the optical fiber 176. In sensor configuration 248, instead of doing a reflection mode measurement, U-bent optical fiber 176 allows for a transmission mode measurement. The bending may allow for some useful optical effects.

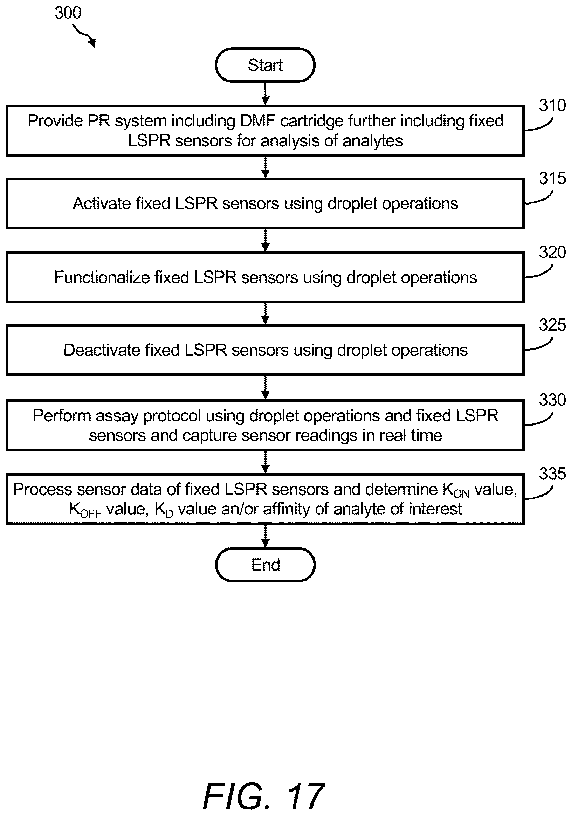

FIG. 17 illustrates a flow diagram of an embodiment of a method 300 of using DMF cartridge 110, fixed LSPR sensing processes, and droplet operations for analysis of analytes. By way of example, method 300 describes a process for a carboxyl group (COOH)-based DMF cartridge 110. However, reactive surface groups may include, for example, COOH, Streptavidin, thiol, protein A, Nitrilotriacetic acid (NTA), and others. Method 300 may include, but is not limited to, the following steps.

At a step 310, PR system 100 may be provided that includes DMF cartridge 110, wherein fixed LSPR sensing processes (e.g., fixed LSPR sensors 136) can be used for the analysis of analytes.

At a step 315, the fixed LSPR sensors 136 can be activated using droplet operations. For example, "activation" may include an amine coupling step in which the COOH functional surface coating on the LSPR sensors 136 is converted into an active ester. For example, an activation buffer droplet that has EDC/NHS therein may be transported using droplet operations to a certain fixed LSPR sensor 136. EDC is 1-Ethyl-3-(3-dimethylaminopropyl)-carbodiimide. NHS is N-hydroxysuccinimide. The activation buffer droplet with the EDC/NHS therein may reside at the fixed LSPR sensor 136 for a period of time. The EDC/NHS solution reacts with the COOH sites on the fixed LSPR sensor 136 and turns them into active functional groups that can covalently bind to any amine group on the ligand. In so doing, the fixed LSPR sensor 136 may be activated.

At a step 320, the fixed LSPR sensors 136 may be functionalized using droplet operations. For example, a buffer droplet that has ligands therein may be transported using droplet operations to a certain fixed LSPR sensor 136. The buffer droplet with the ligands therein may reside at the fixed LSPR sensor 136 for a period of time. In so doing, the fixed LSPR sensor 136 may be functionalized.

At a step 325, the fixed LSPR sensors 136 may be deactivated using droplet operations. Deactivation may be performed to convert any remaining active binding sites on the LSPR sensors 136 into non-active sites. For example, a "blocking" solution, such as ethanolamine, may be used to react with any remaining COOH site and deactivate them. For example, a droplet of ethanolamine may be transported using droplet operations to a certain fixed LSPR sensor 136. The ethanolamine droplet may reside at the fixed LSPR sensor 136 for a period of time. In so doing, the fixed LSPR sensor 136 may be deactivated.

At a step 330, the assay protocol may be performed using droplet operations in DMF cartridge 110 and using the fixed LSPR sensing processes. Additionally, sensor readings may be captured in real time. For example, using illumination source 154 and optical measurement device 156, the LSPR signal from a certain fixed LSPR sensor 136 may be captured in real time while running the assay protocol. For example, an assay protocol may be performed in which the sample droplet (e.g., sample droplet 140) is transported using droplet operations to a certain fixed LSPR sensor 136 and readings for the association phase may be recorded. Next, a buffer droplet may be transported using droplet operations to the fixed LSPR sensor 136 and readings for the dissociation phase may be recorded. Next, a different concentration of the analyte (usually 3X the previous one) can be transported using droplet operations the fixed LSPR sensor 136 and the above may be repeated. This is typically done for at least three analyte concentrations, which may facilitate performing the kinetic analysis. Generally, DMF operations of this step will autonomously perform the dilutions required for analysis.

At a step 335, the sensor data from the fixed LSPR sensor 136 may be processed, and the K_{ON} value, K_{OFF} value, and K_{D} value of the analyte of interest may be determined. For example, using controller 150 of PR system 100, the sensor data from the fixed LSPR sensor 136 may be processed by fitting a binding model to the data and using regression to find the K_{ON} value, K_{OFF} value, and K_{D} value of the analyte of interest that best represents the experimental data. This can be accomplished using a data set that includes, for example, the at least three analyte concentrations described in step 330.

Referring now again to FIG. 5A through FIG. 17 that describe fixed LSPR sensing processes in DMF cartridge 110, the rapid motion of the droplet at the fixed LSPR sensor 136 may be absent. While in this scenario it may not be possible to capture the association phase data (i.e., K_{ON} value) and the dissociation phase data (i.e., K_{OFF} value), this scenario may be useful to capture the analyte affinity data (i.e., K_{D} value).

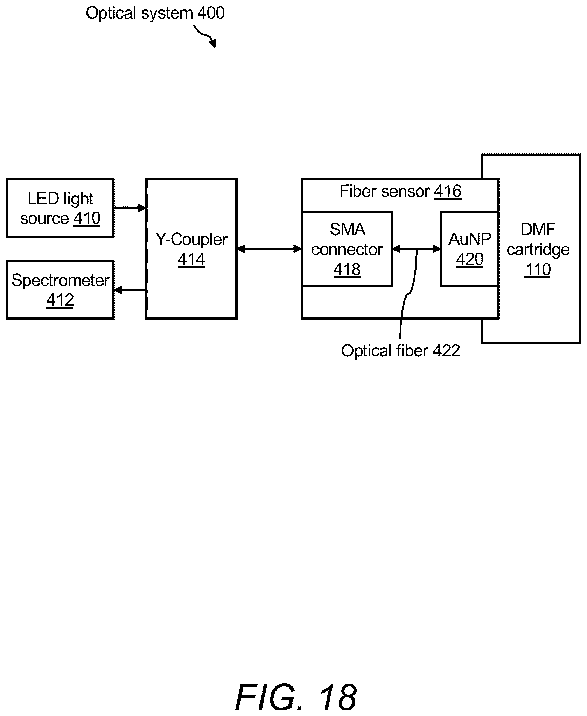

FIG. 18 illustrates a block diagram of an example of an optical system 400 of the presently disclosed PR system 100. In another example of a reflection mode configuration, optical system 400 of the presently disclosed PR system 100 contains a y-coupler that allows the illumination source and reflected light to share a single optical fiber. For example, optical system 400 includes an LED light source 410, a spectrometer 412, and a Y-coupler 414. The optical system 400 also includes a fiber sensor 416 that further includes an SMA connector 418 and a gold nanoparticle (AuNP) tip 420 optically coupled via an optical fiber 422. AuNP tip 420 of fiber sensor 416 is provided in relation to a DMF device, such as DMF cartridge 110. For example, fiber sensor 416 is optically coupled to DMF cartridge 110 via AuNP tip 420. Further, fiber sensor 416 is optically coupled to Y-coupler 414 via SMA connector 418.

In optical system 400, Y-coupler 414 allows LED light source 410 (the illumination source) and reflected light from AuNP tip 420 to share a single optical fiber 422. The advantage of this example is that the illumination source (e.g., LED light source 410) and sensor (e.g., spectrometer 412) are inherently aligned to the activated fiber tip (e.g., AuNP tip 420). For example, a Y-coupler 414 with a 90:10 split can be used with the 10%-side connected to LED light source 410 and the 90%-side connected to spectrometer 412. LED light source 410 then passes through Y-coupler 414 to fiber sensor 416 with gold nanoparticles (e.g., AuNP tip 420) inside the DMF device. This light reflects off the layer of nanoparticles, and certain wavelengths are absorbed according to the SPR signal. The reflected light is then passed through Y-coupler 414 to spectrometer 412 for analysis.

### In-solution LSPR sensing in DMF

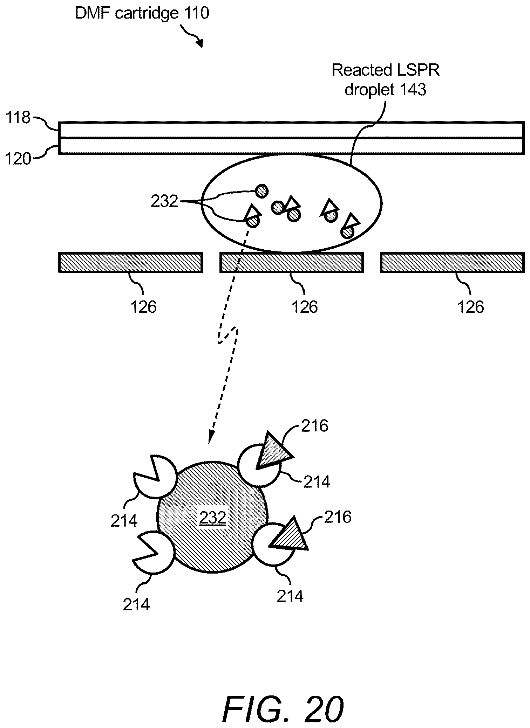

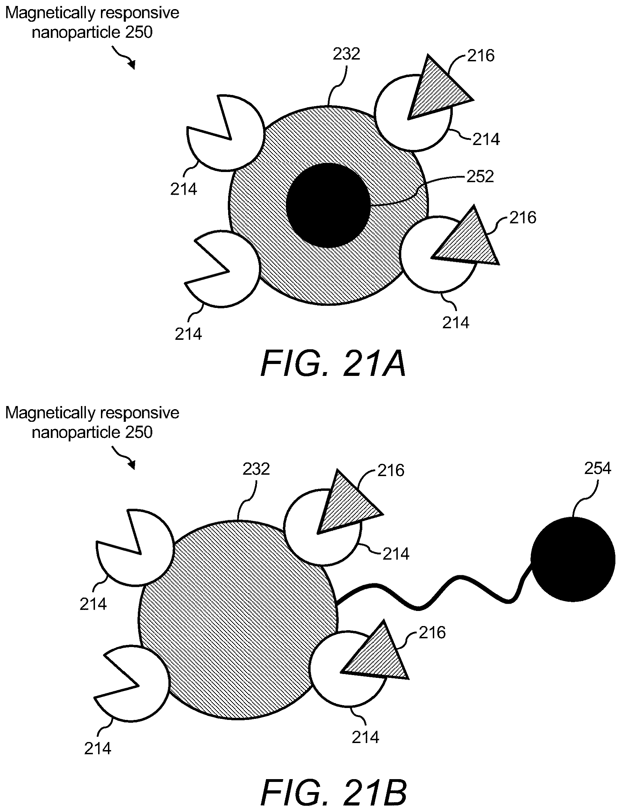

"In-solution LSPR sensing" may refer to any LSPR sensing processes that occur in solution, such as any LSPR sensing processes that occur in the droplets themselves. Below, FIG. 19 and FIG. 20 show an embodiment of a general in-solution LSPR sensing process, while FIG. 21A through FIG. 27 show specific embodiments of in-solution LSPR sensing processes.

FIG. 19 and FIG. 20 illustrate side views of a portion of an embodiment of a DMF cartridge 110 and an embodiment of a general in-solution LSPR sensing process, which is an example of in-solution LSPR sensing 112. In this example, an LSPR droplet 142 may be provided in DMF cartridge 110. LSPR droplet 142 may comprise an aqueous droplet that contains a plurality of nanoparticles 232 suspended therein. Nanoparticles 232 can be the metal nanoparticles, such as gold and/or silver nanoparticles, as described hereinabove with reference to FIG. 3 and FIG. 4. Each of the nanoparticles 232 may be functionalized with capture molecules 214 (e.g., ligands) to create an LSPR effect. In LSPR sensor 136, nanoparticles 232 may be immobilized on a surface. However, here nanoparticles 232 may not be immobilized on a surface, rather nanoparticles 232 may be suspended in solution; such as suspended in LSPR droplet 142.

FIG. 19 shows LSPR droplet 142 along with a separate sample droplet 140. Again, sample droplet 140 may be an aqueous droplet (e.g., a droplet of analyte solution) that contains a plurality of target analytes 216 suspended therein. In this example, target analytes 216 in sample droplet 140 may comprise the binding partner to capture molecules 214 (e.g., ligands) on the nanoparticles 232 in the LSPR droplet 142. Referring now to FIG. 20, in this general in-solution LSPR sensing process, LSPR droplet 142 and sample droplet 140 may be merged and/or mixed using droplet operations and thereby forming a reacted LSPR droplet 143. In so doing, target analytes 216 can bind to capture molecules 214 (not shown) on the nanoparticles 232 due to the LSPR effect of the nanoparticles 232.

Generally, in this process, the flux of the molecules to the surface of the nanoparticles 232 can be created by rapidly mixing the droplets using droplet operations. For example, droplet operations may be used to affect a rapid circular motion (like shown in FIG. 7 and FIG. 9) and/or a rapid back and forth oscillating motion (like shown in FIG. 5) atop the droplet operations electrodes 126. In so doing, binding events may be facilitated in reacted LSPR droplet 143. The binding of the analyte in reacted LSPR droplet 143 causes a shift in the LSPR wavelength. Accordingly, the optical measurement system of PR system 100 can be used to capture the real-time kinetic measurements (e.g., the association phase (i.e., K_{ON} value), the dissociation phase (i.e., K_{OFF} value), and the analyte affinity (i.e., K_{D} value)) of the target analytes 216 to the capture molecules 214 (not shown) on the nanoparticles 232.

FIG. 21A and FIG. 21B illustrate side views of embodiments of magnetically responsive nanoparticles 250 that can be used in the in-solution LSPR sensing processes of the presently disclosed PR system 100 and DMF cartridge 110. "Magnetically responsive" means responsive to a magnetic field. "Magnetically responsive nanoparticles" may include or comprise magnetically responsive materials. Examples of magnetically responsive materials include, for example, paramagnetic materials, ferromagnetic materials, ferrimagnetic materials, and metamagnetic materials. Examples of suitable paramagnetic materials include, but are not limited to, iron, nickel, and cobalt, as well as metal oxides, such as Fe3O4, BaFe12O19, CoO, NiO, Mn2O3, Cr2O3, and CoMnP.

In one example and referring now to FIG. 21A, a magnetically responsive nanoparticle 250 may be formed by a nanoparticle 232 that has a magnetically responsive core element 252 embedded therein. Magnetically responsive core element 252 can be, for example, a magnetically responsive particle. Further, in magnetically responsive nanoparticle 250, nanoparticle 232 may include more than one magnetically responsive core element 252.

In another example and referring now to FIG. 21B, a separate magnetically responsive element 254 may be tethered to nanoparticle 232 to form a magnetically responsive nanoparticle 250. For example, magnetically responsive element 254 can be a magnetically responsive particle that is linked to nanoparticle 232 using physical or chemical coupling. In one example, magnetically responsive element 254 may be coupled to nanoparticle 232 using a linker. Further, more than one magnetically responsive element 254 can be coupled to nanoparticle 232, or more than one nanoparticle 232 can be coupled to one magnetically responsive element. Examples of using magnetically responsive nanoparticles 250 for in-solution LSPR sensing are shown and described hereinbelow with reference to FIG. 19 through FIG. 27.

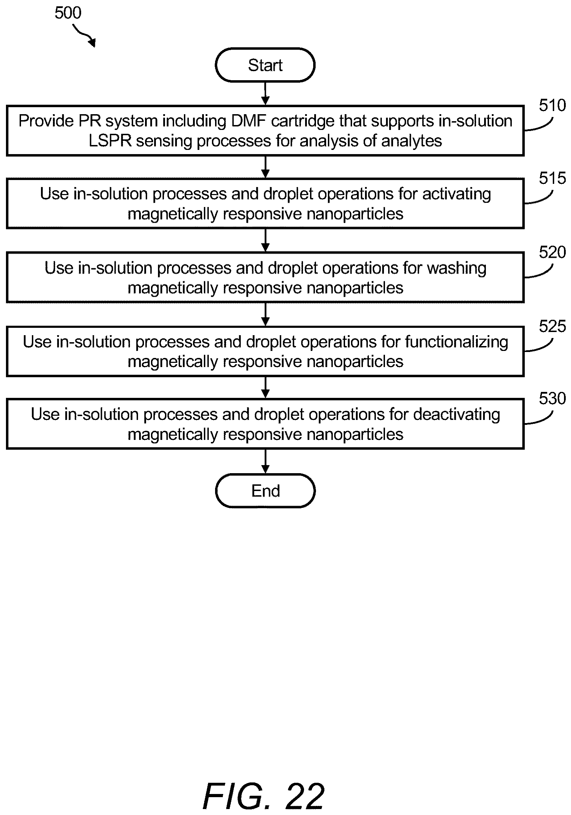

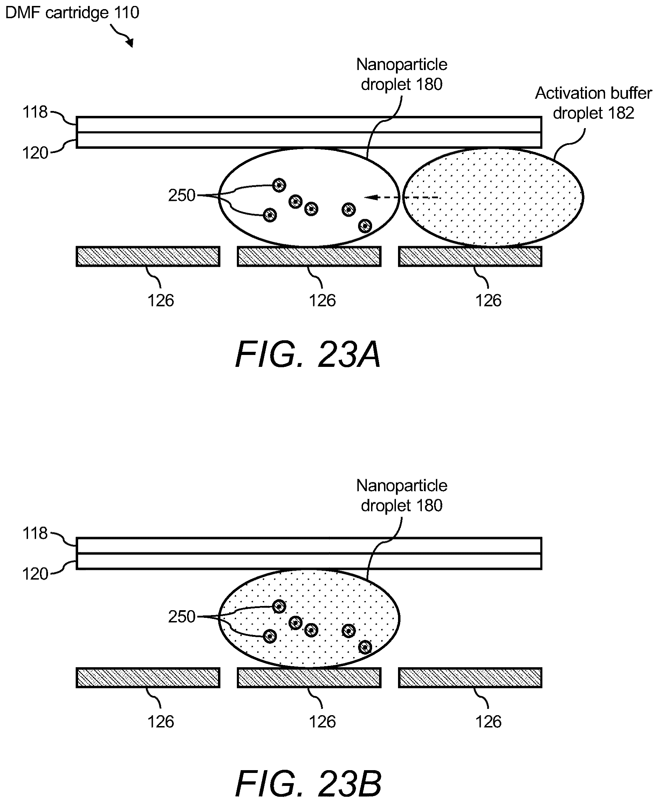

FIG. 22 illustrates a flow diagram of an embodiment of an in-solution process 500 of functionalizing magnetically responsive nanoparticles 250 using droplet operations. By way of example, in-solution process 500 describes a process for a COOH-based DMF cartridge 110. However, reactive surface groups may include, for example, COOH, Streptavidin, and NTA. Additionally, FIG. 23A through FIG. 23F illustrate pictorially certain steps of an embodiment of an in-solution process 500 and therefore FIG. 23A through FIG. 23F may be referenced in certain steps of in-solution process 500. In-solution process 500 may include, but is not limited to, the following steps.

At a step 510, PR system 100 may be provided that includes DMF cartridge 110 that supports in-solution LSPR sensing processes that use droplet operations for analysis of analytes.

At a step 515, in-solution processes and droplet operations may be used for activating magnetically responsive nanoparticles 250. For example, "activation" may comprise an amine coupling step in which the COOH functional surface coating on the magnetically responsive nanoparticles 250 is converted into an active ester. For example and referring now to FIG. 23A, a nanoparticle droplet 180 may be provided in DMF cartridge 110. Nanoparticle droplet 180 may be a solution containing magnetically responsive nanoparticles 250 that have the COOH coating thereon. Next and still referring now to FIG. 23A, an activation buffer droplet 182 may be provided in DMF cartridge 110. The activation buffer droplet 182 includes EDC/NHS. Next and referring now to FIG. 23B, nanoparticle droplet 180 and activation buffer droplet 182 may be combined or merged using droplet operations. In so doing, the nanoparticle droplet 180 may be activated. For example, by merging nanoparticle droplet 180 and activation buffer droplet 182 for a period of time the magnetically responsive nanoparticles 250 in nanoparticle droplet 180 may be activated. The magnetically responsive nanoparticles 250 in nanoparticle droplet 180 are now prepared for ligand functionalization.

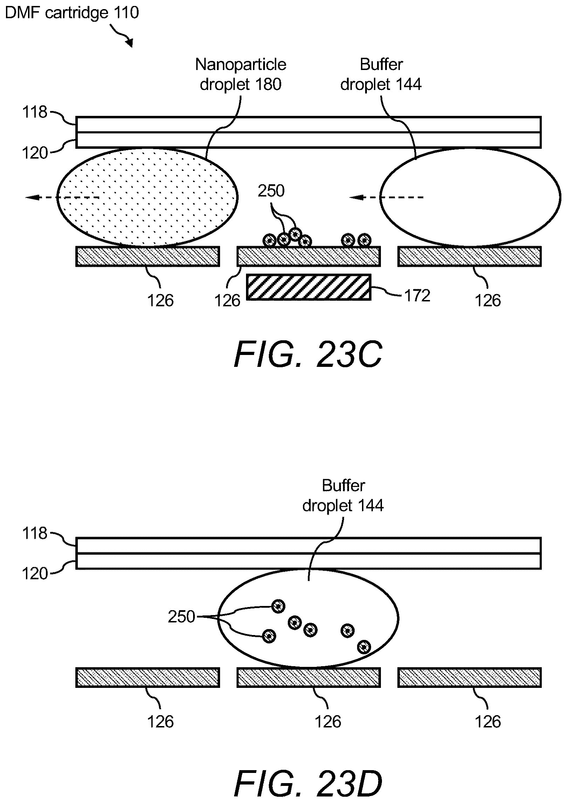

At a step 520, in-solution processes and droplet operations may be used for washing magnetically responsive nanoparticles 250. For example and referring now to FIG. 23C, a magnet (e.g., magnet 172, which is a permanent magnet or electromagnet) may be used to immobilize the magnetically responsive nanoparticles 250 in nanoparticle droplet 180 atop a certain droplet operations electrode 126. Next, nanoparticle droplet 180 may be transported away using droplet operations and leaving the magnetically responsive nanoparticles 250 immobilized atop the droplet operations electrode 126. "Immobilize" with respect to magnetically responsive nanoparticles, may mean that the nanoparticles are substantially restrained in position atop a droplet operations electrode in a DMF cartridge such that forces acting on the nanoparticles resulting from fluid flow relative to the nanoparticles is insufficient to dislodge the nanoparticles from the restrained position. Next and referring now to FIG. 23C and FIG. 23D, a buffer droplet 144 may be transported using droplet operations to the site of the immobilized magnetically responsive nanoparticles 250. Buffer droplets 140 can be passed multiple times over the immobilized magnetically responsive nanoparticles 250 as needed to wash the structures.

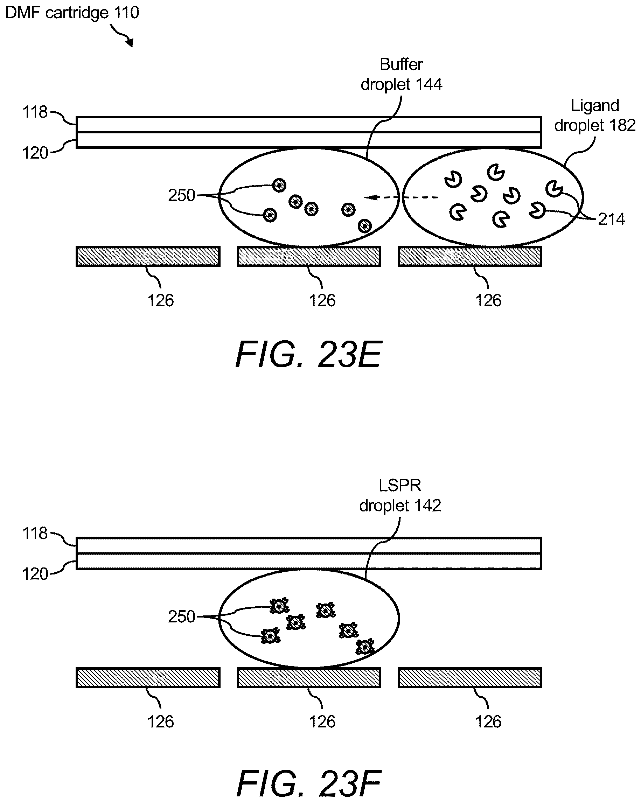

At a step 525, in-solution processes and droplet operations may be used for functionalizing magnetically responsive nanoparticles 250. Next and referring now to FIG. 23E, the magnetically responsive nanoparticles 250 may be released into buffer droplet 144. The buffer droplet 144 may then combined or merged with, for example, a ligand droplet 182, wherein ligand droplet 182 comprises capture molecules or ligands 214. In so doing, the LSPR droplet 142 may be formed and the magnetically responsive nanoparticles 250 therein are functionalized. For example, by merging buffer droplet 144 (with the magnetically responsive nanoparticles 250 therein) and ligand droplet 182 for a period of time the ligands 214 may attach to the magnetically responsive nanoparticles 250 and form LSPR droplet 142. Then, certain washing operations may occur by exchanging multiple buffer droplets.

At a step 530, in-solution processes and droplet operations may be used for deactivating magnetically responsive nanoparticles 250. Deactivation may be performed to convert any remaining active binding sites on the magnetically responsive nanoparticles 250 in LSPR droplet 142 into non-active sites. For example, a "blocking" solution, such as ethanolamine, may be used to react with any remaining COOH sites and deactivate them. For example, a droplet of ethanolamine may be combined or merged with LSPR droplet 142. For example, by merging the LSPR droplet 142 (with the magnetically responsive nanoparticles 250 therein) and the ethanolamine droplet for a period of time, the LSPR droplet 142 may be deactivated. Then, certain washing operations may occur by exchanging multiple buffer droplets.

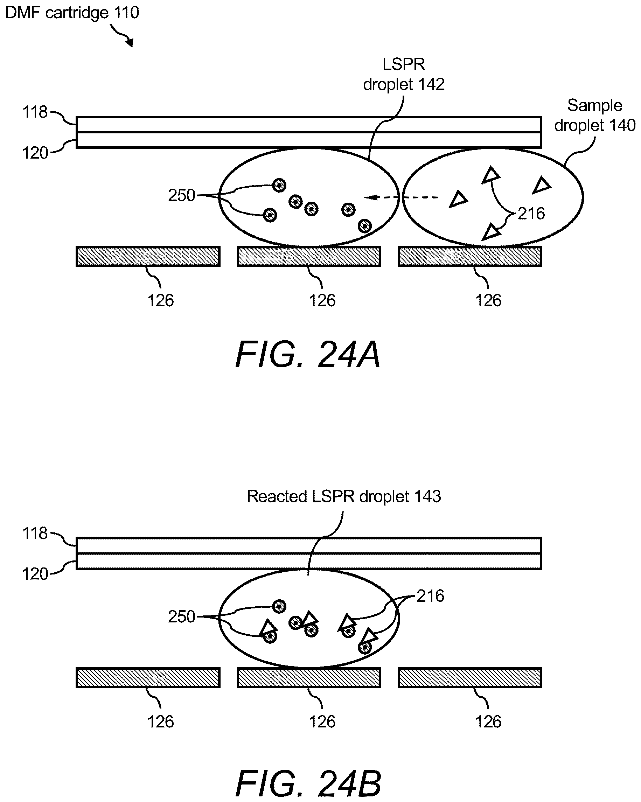

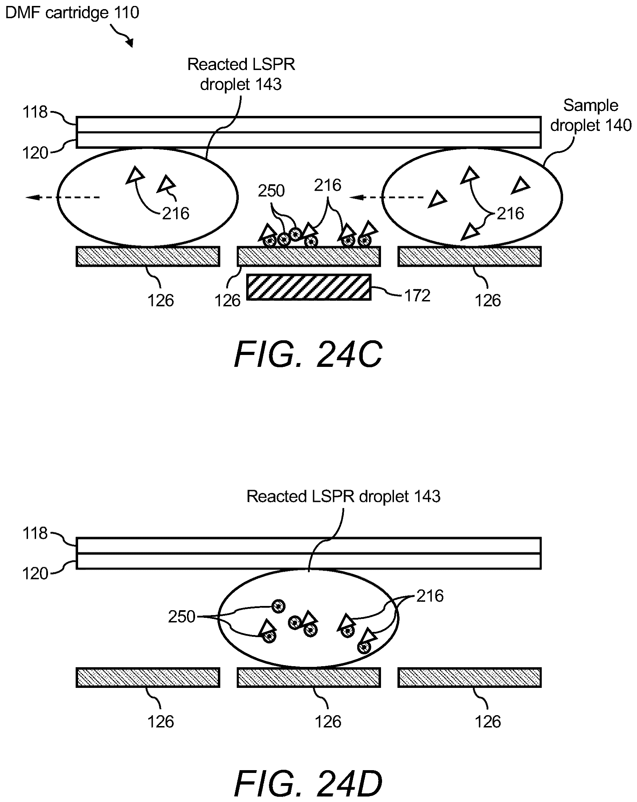

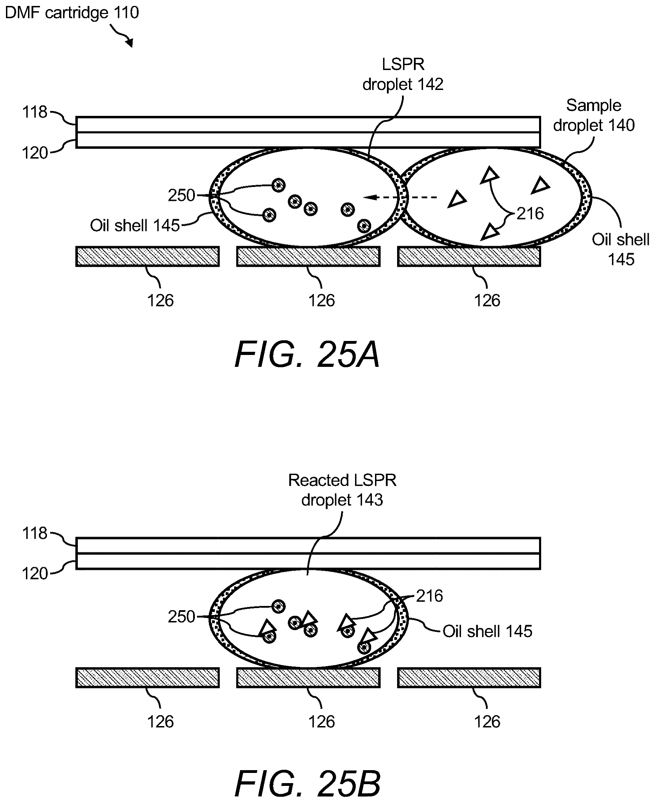

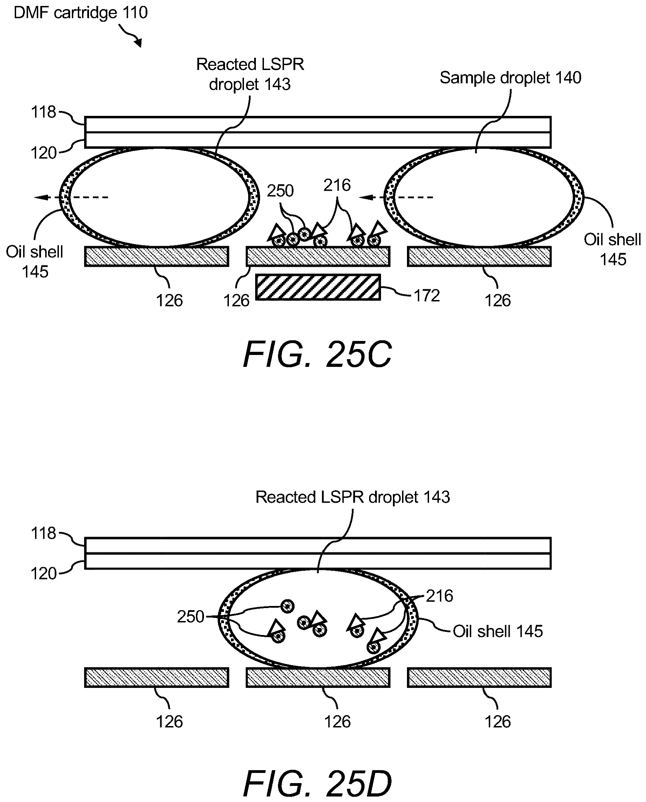

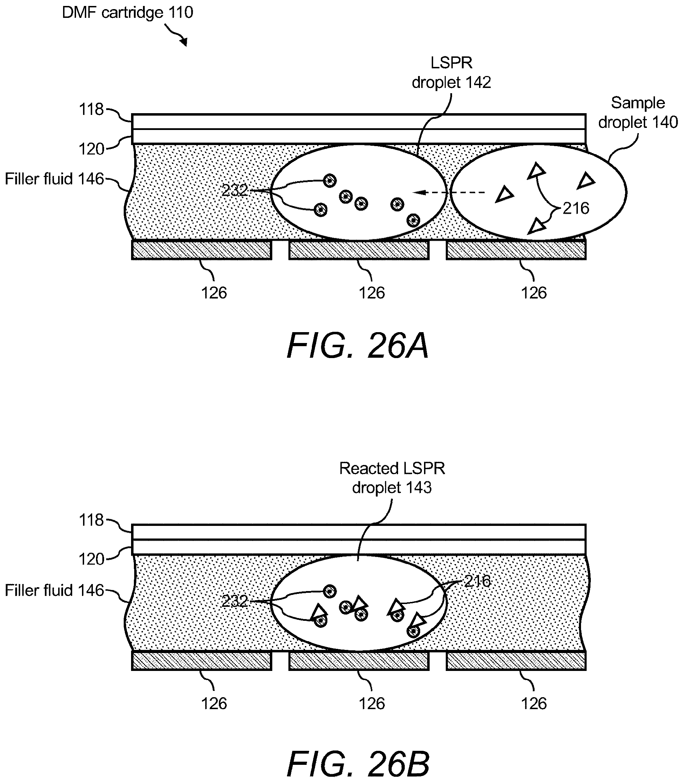

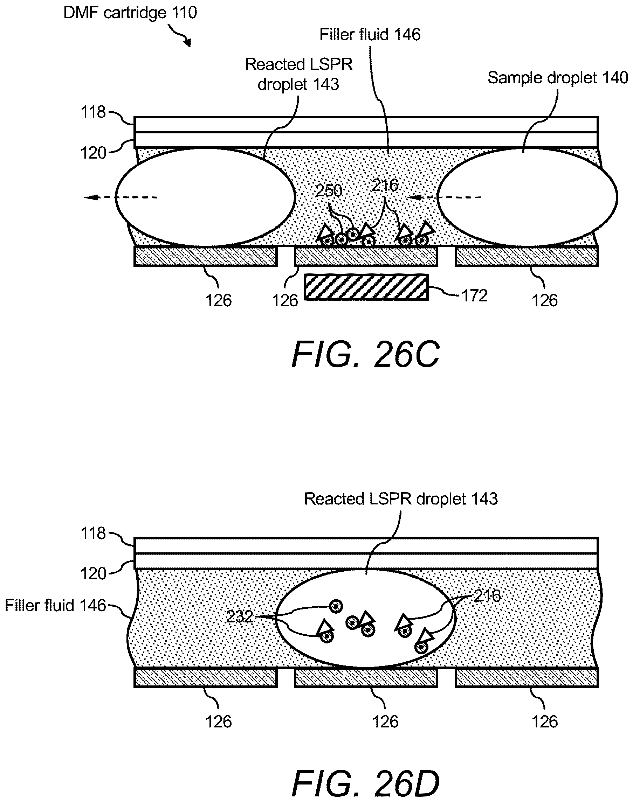

FIG. 24A, FIG. 24B, FIG. 24C, and FIG. 24D; FIG. 25A, FIG. 25B, FIG. 25C, and FIG. 25D; and FIG. 26A, FIG. 26B, FIG. 26C, and FIG. 26D illustrate embodiments of in-solution LSPR sensing processes in DMF cartridge 110 using the magnetically responsive nanoparticles and droplet operations. For example, FIG. 24A, FIG. 24B, FIG. 24C, and FIG. 24D show an in-solution LSPR sensing process in DMF cartridge 110 wherein droplet operations may occur in air. FIG. 25A, FIG. 25B, FIG. 25C, and FIG. 25D show an in-solution LSPR sensing process in DMF cartridge 110 wherein droplet operations may occur in air with oil-covered droplets. FIG. 26A, FIG. 26B, FIG. 26C, and FIG. 26D show an in-solution LSPR sensing process in DMF cartridge 110 wherein droplet operations may occur in oil (or filler fluid).

With reference now to FIG. 24A, an LSPR droplet 142 may be provided in DMF cartridge 110. LSPR droplet 142 may have been prepared, for example, according to in-solution process 500 shown in FIG. 22. Further, sample droplet 140 may be provided that has target analytes 216 therein. With reference now to FIG. 24B, LSPR droplet 142 and sample droplet 140 may be combined or merged using droplet operations and rapidly mixed. For example, the droplets are rapidly mixed using droplet operations to affect rapid circular motion (like shown in FIG. 7 and FIG. 9) and/or rapid back and forth oscillating motion (like shown in FIG. 5) atop the droplet operations electrodes. In so doing, binding events may occur on the magnetically responsive nanoparticles 250 in LSPR droplet 142, which is now called reacted LSPR droplet 143.

In this process, the sample droplet 140 may need to be swapped out periodically with a fresh sample droplet 140 in order to avoid depletion effects in the data (i.e., the concentration of an analyte in the bulk phase decreasing because it is binding to the surface). Alternatively, the swapping out procedure could be triggered through real-time monitoring of some measurable property of the droplet or its contents. For example and referring now to FIG. 24C, this can be done rapidly using magnets to immobilize magnetically responsive nanoparticles 250 and bring in a new analyte drop (e.g., sample droplet 140). For example, magnet 172 may be used to immobilize the magnetically responsive nanoparticles 250 in reacted LSPR droplet 143 atop a certain droplet operations electrode 126. Next, reacted LSPR droplet 143 may be transported away using droplet operations, leaving the magnetically responsive nanoparticles 250 immobilized atop the droplet operations electrode 126. Referring now to both FIG. 24C and FIG. 24D, a sample droplet 140 may be transported using droplet operations to the site of the immobilized magnetically responsive nanoparticles 250. Then, magnetically responsive nanoparticles 250 may be released back into suspension. During this process, optical measurements can be taken in real-time to capture the affinity data and the association phase data.

Referring now again to both FIG. 24C and FIG. 24D, once a set amount of time has passed, sample droplet 140 may be swapped out with a buffer droplet 144, and optical measurements can be taken in real-time to capture the dissociation phase data. For example, again, magnet 172 may be used to immobilize the magnetically responsive nanoparticles 250 in reacted LSPR droplet 143 atop a certain droplet operations electrode 126. Next, reacted LSPR droplet 143 may be transported away using droplet operations and leaving the magnetically responsive nanoparticles 250 immobilized atop the droplet operations electrode 126. Then, buffer droplet 144 may be transported using droplet operations to the site of the immobilized magnetically responsive nanoparticles 250. Then, magnetically responsive nanoparticles 250 may be released back into suspension, and the dissociation phase may be measured. Multiple buffer droplets may be exchanged to ensure the analyte does not accumulate.

The process shown in FIG. 25A, FIG. 25B, FIG. 25C, and FIG. 25D may be substantially the same as the process described hereinabove with reference to FIG. 24A, FIG. 24B, FIG. 24C, and FIG. 24D except that the droplets are oil-covered droplets (e.g., droplets with oil shell 145). In this example, when magnetically responsive nanoparticles 250 are immobilized, they will pass briefly through oil shell 145 and then be resuspended into the aqueous portion of the droplets. Because the surface of magnetically responsive nanoparticles 250 is generally hydrophilic, there tends to be an aqueous layer on the surface of magnetically responsive nanoparticles 250 which may resist oil contamination and may help promote a reliable reading. Further, this configuration can be used to minimize the amount of time that the magnetically responsive nanoparticles 250 are exposed to the oil phase.

Further, the process shown in FIG. 26A, FIG. 26B, FIG. 26C, and FIG. 26D may be substantially the same as the process described hereinabove with reference to FIG. 24A, FIG. 24B, FIG. 24C, and FIG. 24D except that droplet operations may occur in oil (e.g., filler fluid 146)). In this example, when magnetically responsive nanoparticles 250 are immobilized, they will transition from the full oil environment of DMF cartridge 110 to the fully aqueous environment of the droplets. Again, because the surface of magnetically responsive nanoparticles 250 is generally hydrophilic, there tends to be an aqueous layer on the surface of magnetically responsive nanoparticles 250 which helps resists oil contamination and helps promote a reliable reading.

Further, in the processes shown in FIG. 23 through FIG. 26D, the position of magnet 172 (e.g., a permanent magnet or electromagnet) is not limited to near bottom substrate 116 only. Magnet 172 can be positioned elsewhere, such as near top substrate 118 or on the side or edge of DMF cartridge 110.

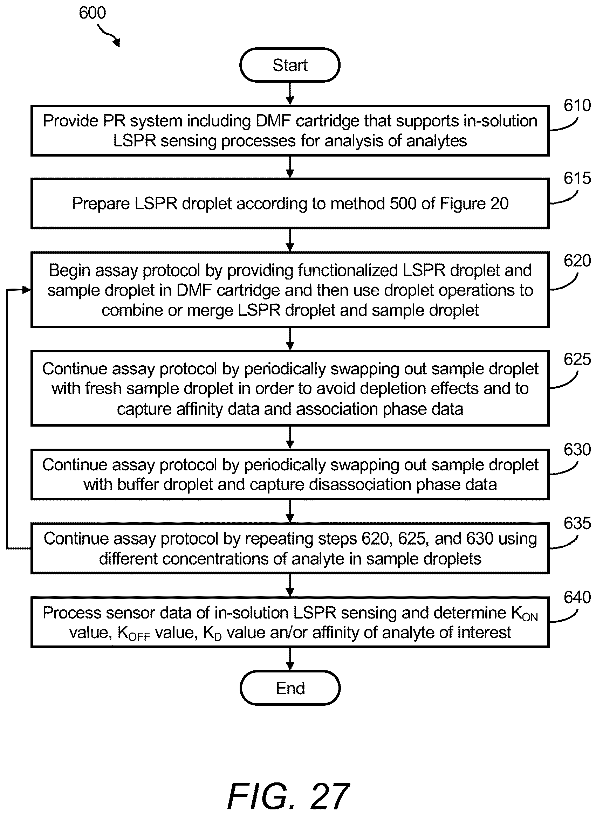

FIG. 27 illustrates a flow diagram of an embodiment of a method 600 of using DMF cartridge 110, in-solution LSPR sensing processes, and droplet operations for analysis of analytes. By way of example, method 600 may include a process for a COOH-based DMF cartridge 110. However, reactive surface groups may include, for example, COOH, Streptavidin, and NTA. Method 600 reflects each of the processes shown in FIG. 24A, FIG. 24B, FIG. 24C, and FIG. 24D; FIG. 25A, FIG. 25B, FIG. 25C, and FIG. 25D; and FIG. 26A, FIG. 26B, FIG. 26C, and FIG. 26D. Method 600 may include, but is not limited to, the following steps.

At a step 610, PR system 100 may be provided that includes DMF cartridge 110 that supports in-solution LSPR sensing processes that use droplet operations for analysis of analytes.

At a step 615, droplet operations may be used in DMF cartridge 110 to prepare LSPR droplets for analysis of analytes using in-solution LSPR sensing. For example, LSPR droplets 142 may be functionalized according to in-solution process 500 shown and described with reference to FIG. 22.

At a step 620, the method 600 may include beginning assay protocol by providing the functionalized LSPR droplet and the sample droplet in the DMF cartridge and then using droplet operations to combine or merge the LSPR droplet and the sample droplet. For example and referring now to FIG. 24A, an LSPR droplet 142 may be provided in DMF cartridge 110. Further, sample droplet 140 may be provided that has target analytes 216 therein. With reference now to FIG. 24B, LSPR droplet 142 and sample droplet 140 may be combined or merged using droplet operations and rapidly mixed. For example, the droplets may be rapidly mixed using droplet operations to affect rapid circular motion and/or rapid back and forth oscillating motion atop the droplet operations electrodes. In so doing, binding events occur on the magnetically responsive nanoparticles 250 in LSPR droplet 142, which is now called reacted LSPR droplet 143.

At a step 625, the method 600 may comprise continuing the assay protocol by periodically swapping out the sample droplet with a fresh sample droplet in order to avoid depletion effects and to capture the affinity data and the association phase data. For example and referring now to FIG. 24C, periodically (e.g., every few seconds) the sample droplet 140 may need to be switched out with a fresh sample droplet 140 in order to reduce depletion effects in the data. For example, magnet 172 may be used to immobilize the magnetically responsive nanoparticles 250 in reacted LSPR droplet 143 atop a certain droplet operations electrode 126. Next, reacted LSPR droplet 143 may be transported away using droplet operations and leaving the magnetically responsive nanoparticles 250 immobilized atop the droplet operations electrode 126. Referring now to both FIG. 24C and FIG. 24D, a sample droplet 140 may be transported using droplet operations to the site of the immobilized magnetically responsive nanoparticles 250. Then, magnetically responsive nanoparticles 250 may be released back into suspension. During this process, optical measurements can be taken in real-time to capture the affinity data and the association phase data.

At a step 630, the method 600 may continue assay protocol by periodically swapping out the sample droplet with a buffer droplet and capture the dissociation phase data. For example and referring now again to both FIG. 24C and FIG. 24D, once a given amount of time has passed or the signal is monitored to determine when it is time to swap liquids, sample droplet 140 may be swapped out with a buffer droplet 144, and optical measurements can be taken in real-time to capture the dissociation phase data. For example, again, magnet 172 may be used to immobilize the magnetically responsive nanoparticles 250 in reacted LSPR droplet 143 atop a certain droplet operations electrode 126. Next, reacted LSPR droplet 143 may be transported away using droplet operations, leaving the magnetically responsive nanoparticles 250 immobilized atop the droplet operations electrode 126. Then, buffer droplet 144 may be transported using droplet operations to the site of the immobilized magnetically responsive nanoparticles 250. Then, magnetically responsive nanoparticles 250 may be released back into suspension. Again, buffer droplets 140 can be passed multiple times over the immobilized magnetically responsive nanoparticles 250 as needed to wash the structures.

At a step 635, the method 600 may continue assay protocol by repeating steps 620, 625, and 630 using different concentrations of analyte (usually 3X the previous one) in sample droplets 140. This is typically done for at least three analyte concentrations, which may be used to perform the kinetic analysis.

At a step 640, the sensor data from the LSPR droplets 142 of the in-solution LSPR sensing processes may be processed, and the K_{ON} value, K_{OFF} value, K_{D} value, and/or affinity of the analyte of interest is determined. For example, using controller 150 of PR system 100, the sensor data from the LSPR droplets 142 may be processed by fitting a binding model to the data and using a regression to find the K_{ON} value, K_{OFF} value, K_{D} value, and/or affinity of the analyte of interest that best represents the experimental data. This can be accomplished using a data set that includes, for example, the at least three analyte concentrations described in step 635.

A feature of method 600 that uses in-solution LSPR sensing processes and droplet operations for analysis of analytes may include the fact large numbers (e.g., hundreds) of droplets can be processed simultaneously using PR system 100 and DMF cartridge 110. Additionally, the in-solution LSPR sensing processes in DMF cartridge 110 may allow one to easily test against multiple analytes and/or concentrations with high consistency because LSPR droplets 142 functionalized according to in-solution process 500 of FIG. 22 can be split into multiple drops and processed separately.

Further, another feature of the in-solution process 500 of FIG. 22 and method 600 of FIG. 27 may be that the chemical preparations of the droplets can be done using in-solution process 500 of FIG. 22, then using method 600 of FIG. 27 the kinetic measurements may be performed while the magnetically responsive nanoparticles 250 are immobilized.

Further, another feature of in-solution process 500 of FIG. 22 and method 600 of FIG. 27 may be that various analyses may use precisely the same device with only a change of analytes/activation.

Further, another feature of in-solution process 500 of FIG. 22 and method 600 of FIG. 27 may be that this design facilitates autonomous optimization of the immobilization chemistry for the specific analyte.

Further, another feature of in-solution process 500 of FIG. 22 and method 600 of FIG. 27 may be that the nanoparticles 250 in the droplets can be easily washed. This may facilitate surface chemistry and immobilization of ligands to be done directly on the nanostructures within DMF cartridge 110. This feature may allow complex samples like blood to be processed.

With reference now again to FIG. 19 through FIG. 27 that describe in-solution LSPR sensing processes in DMF cartridge 110, the rapid motion of the droplets may be absent. While in this scenario it may not be possible to capture the association phase data (i.e., K_{ON} value) and the dissociation phase data (i.e., K_{OFF} value), this scenario may be useful to capture the analyte affinity data (i.e., K_{D} value). Additionally, the K_{ON} and K_{OFF} can be inferred via additional equations modeling diffusion.

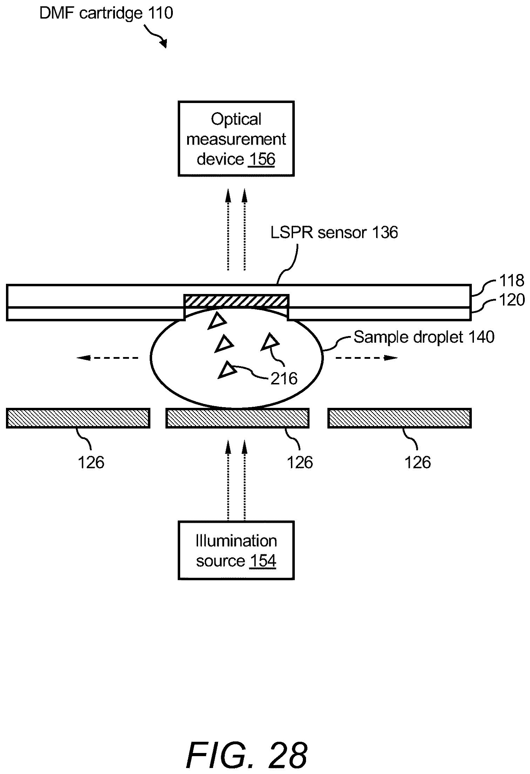

Referring again to FIG. 1 through FIG. 27, various configurations of the optical measurement system of PR system 100 can be used. For example, FIG. 28 through FIG. 32 show examples of different configurations of the optical measurement system. For example, FIG. 28 shows illumination source 154 and optical measurement device 156 configured for operating in transmission mode in relation to fixed LSPR sensor 136 in DMF cartridge 110. In this example, bottom substrate 116, top substrate 118, and droplet operations electrodes 126 may be substantially optically transparent or contain optically transparent windows within the sensing regions.

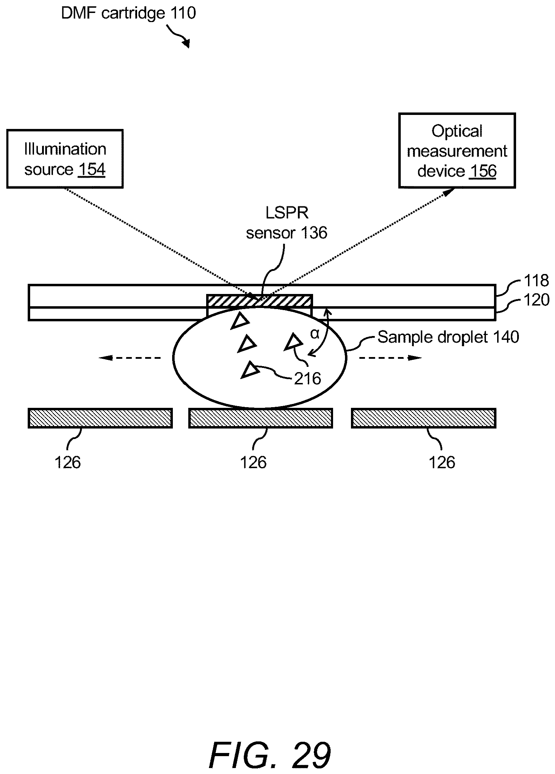

FIG. 29 shows illumination source 154 and optical measurement device 156 configured for operating in reflection mode in relation to fixed LSPR sensor 136 in DMF cartridge 110. When operating in reflection mode, the DMF cartridge 110 may be configured to allow for optical observation from a given side of the DMF cartridge 110. In this regard, either the top substrate 118 or the bottom substrate 116 may be optically transparent or contain optically transparent windows depending on the side of the DMF cartridge 110 the optical measurement system is disposed. Specifically, the portion of the substrate adjacent to the optical measurement system may be optically transparent, and the opposite side may not be optically transparent when operated in reflection mode. Accordingly, in the embodiment depicted in FIG. 29, top substrate 118 may be substantially optically transparent whereas bottom substrate 116 (not shown) and droplet operations electrodes 126 may not be substantially optically transparent.

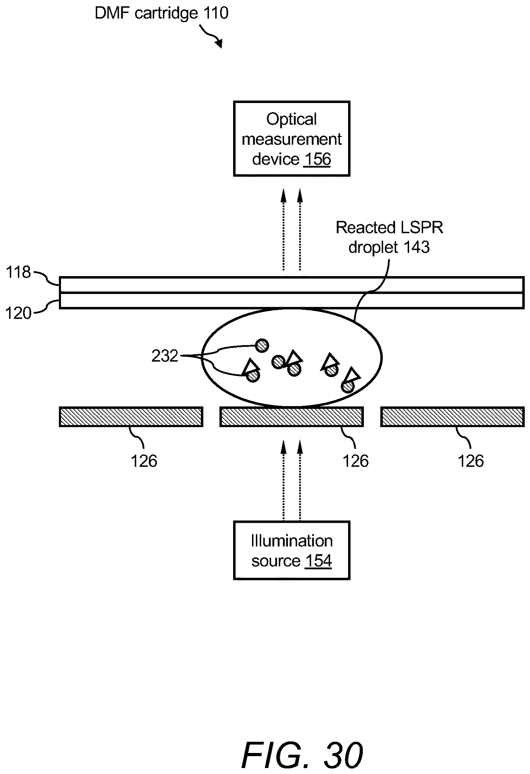

FIG. 30 shows illumination source 154 and optical measurement device 156 configured for operating in transmission mode in relation to in-solution LSPR sensing processes in DMF cartridge 110. In this example, bottom substrate 116, top substrate 118, and droplet operations electrodes 126 are substantially optically transparent.

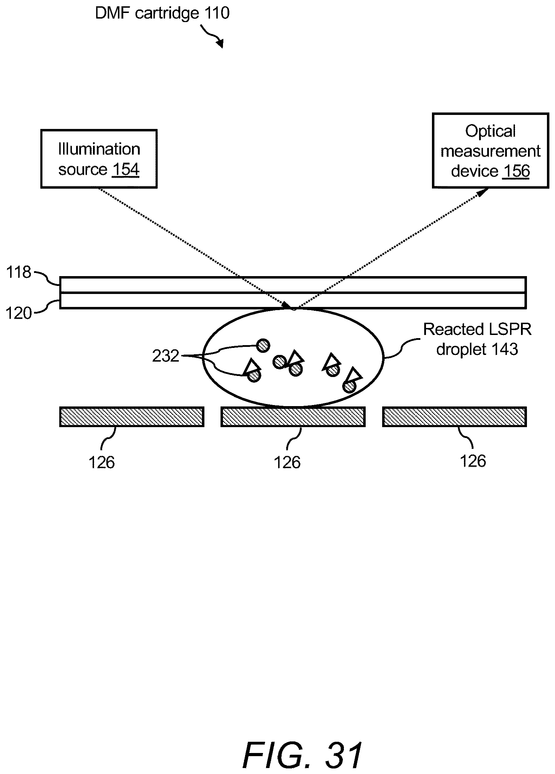

FIG. 31 shows illumination source 154 and optical measurement device 156 configured for operating in reflection mode in relation to in-solution LSPR sensing processes in DMF cartridge 110. As described above, when operated in reflection mode, the substrate adjacent to the optical measurement device 156 may be optically transparent, whereas the side opposite the optical measurement device 156 may not be optically transparent. In the embodiment shown in FIG. 31, top substrate 118 may be substantially optically transparent whereas bottom substrate 116 (not shown) and droplet operations electrodes 126 may not be substantially optically transparent.

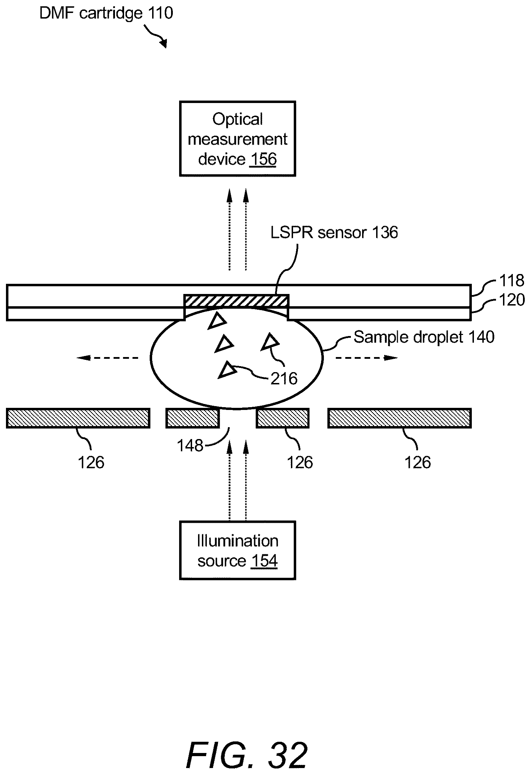

FIG. 32 shows an example of DMF cartridge 110 that may include an optical aperture 148 in relation to the illumination source 154 and/or optical measurement device 156. In this example, optical aperture 148 may be in one of the droplet operations electrodes 126, wherein the droplet operations electrodes 126 may not be substantially optically transparent.

### Example Testing

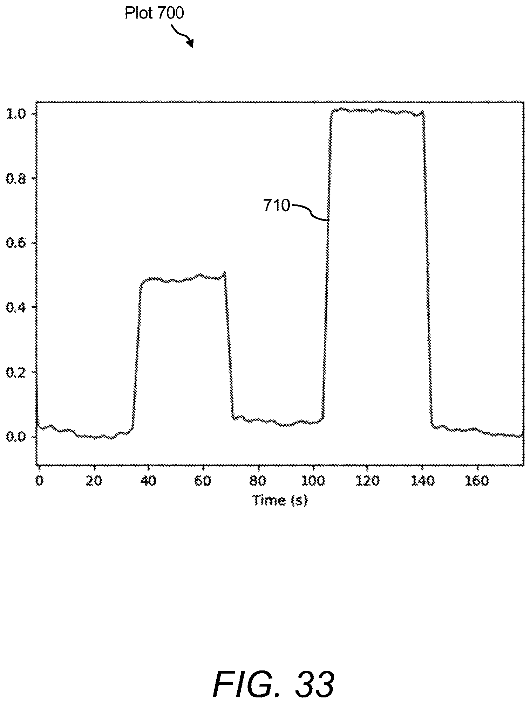

FIG. 33 shows an example of a plot 700 of an SPR response vs. time for first a drop of 1% glycerol in DI water followed by a drop of 2% glycerol in DI water. For example, plot 700 shows an SPR response curve 710. In plot 700, an LSPR sensor on a fiber tip with a COOH functional group was exposed first to deionized water (0-33s), 1% glycerol in deionized water (33S-66s), deionized water (66s-103s), 2% glycerol (103s-140s) and finally deionized water again. The experiment was performed in an oil environment.

This experiment proves 2 points: (1) that the optical fiber has LSPR active gold nanoparticles on its tip. The sensor can detect the refractive index shift from water to both 1% and 2% glycerol. Furthermore, the difference in signal between 1% and 2% is linear as expected; and (2) that the oil environment (a refractive index shift over 30x higher than 2% glycerol) does not interfere with the measurements.

Previously, the point has been made that the generally hydrophilic LSPR sensor retains an aqueous layer that protects the sensor against the oil environment. Despite a 2-second exposure to the oil medium in between each solution exchange, no effect of this exposure is noted in the data. This is due to the short-interaction distance of the LSPR sensor being within the retained aqueous layer.

A proof of concept experiment for the activation of the LSPR optical fiber with ligand was performed. In this proof of principle experiment, a COOH-surfaced LSPR sensor on a fiber was embedded into a DMF device, and a ligand immobilization procedure was performed. This process, described below, consisted of activation of the COOH surface with EDC+NHS, attachment of Protein A to this sensor surface, the introduction of ethanolamine to passivate unreacted surface sites and finally the introduction of human IgG that binds to Protein A to verify the ligand immobilization.

In this experiment, an optical fiber with a COOH-surfaced LSPR sensor on its tip was inserted from the side into a DMF device. The DMF device then performed commands to expose the fiber tip to the following samples. Each step, unless specified otherwise, consisted of dispensing 600 nL of the chemical in question and moving that volume to the fiber tip. During the exposure time, the device oscillated the liquid at a rate of 4 Hz. The following procedure was performed:
1) 10 minutes of phosphate buffered saline (PBS) to condition the sensor surface,
2) 2 minutes of 10 mM hydrochloric acid (HCl) to clean the sensor surface,
3) 5 minutes of PBS,
4) The DMF device dispensed 300 nL of EDC and 300 nL of NHS, mixed the result and exposed the sensor to the mixture for 5 minutes,
5) 3.5 minutes of PBS,
6) 5 minutes of 20 µg/mL Protein A,
7) 2 minutes of PBS to wash off excess Protein A,
8) 5 minutes of ethanolamine to block un-reacted binding sites,
9) 6 minutes of PBS,
10) 5 minutes exposure to 100 nM Human IgG to validate Protein A binding, and
11) 8 minutes of PBS.

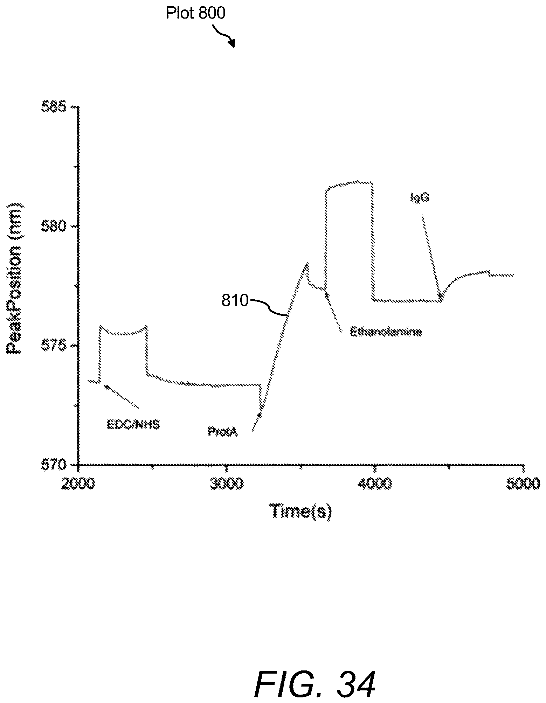

The results of this experiment are shown in FIG. 34. The plot 800 of the peak position for activating a carboxyl-gold optical fiber, binding Protein A, blocking with ethanolamine, and binding IgG. Plot 800 shows a curve 810 where each droplet exchange operated as expected with the binding steps (Protein A, Ethanolamine, IgG) exhibiting traditional binding-curves. This validates the platform as a method of automating SPR sample mixing and analysis.

Another proof of concept experiment for the activation of the LSPR optical fiber with ligand was performed. In this proof of principle experiment, a Protein A-activated LSPR sensor was inserted into a DMF device and introduced to a series of concentrations of analyte with a regeneration step in-between. This allows for the determination of the analyte binding kinetics.

In this experiment, an optical fiber with a Protein A activated LSPR sensor on its tip was inserted from the side into a DMF device. The DMF device then performed commands to expose the fiber tip to the following samples. In this experiment, each step consisted of a 600 nL sample that was oscillated at 10 Hz. The following procedure was run:
1) 10 mins PBS to condition the sensor surface,
2) 2.5 mins 11 nM Human IgG analyte,
3) 12.5 mins PBS to measure release kinetics,
4) 2.5 mins pH 2.0 Glycine/HCl to regenerate the surface,
5) 2.5 mins 33 nM IgG Human IgG analyte,
6) 12.5 mins PBS to measure release kinetics,
7) 2.5 mins pH 2.0 Glycine/HCl to regenerate the surface,
8) 2.5 mins 100 nM IgG Human IgG analyte, and
9) 12.5 mins PBS to measure release kinetics.

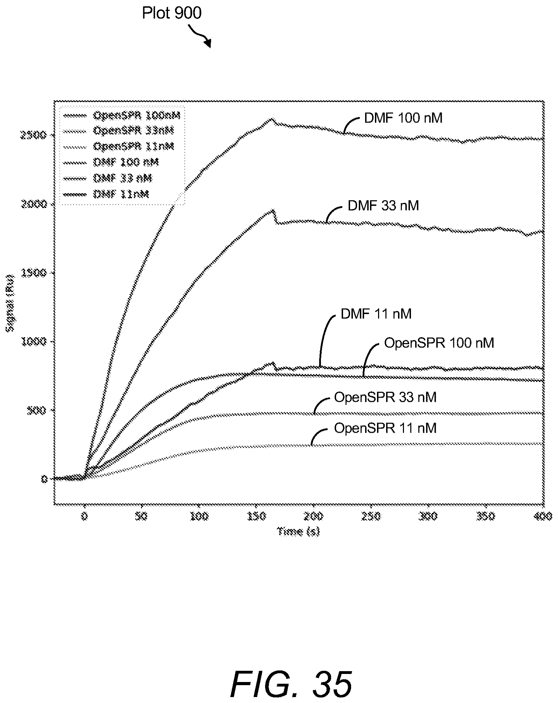

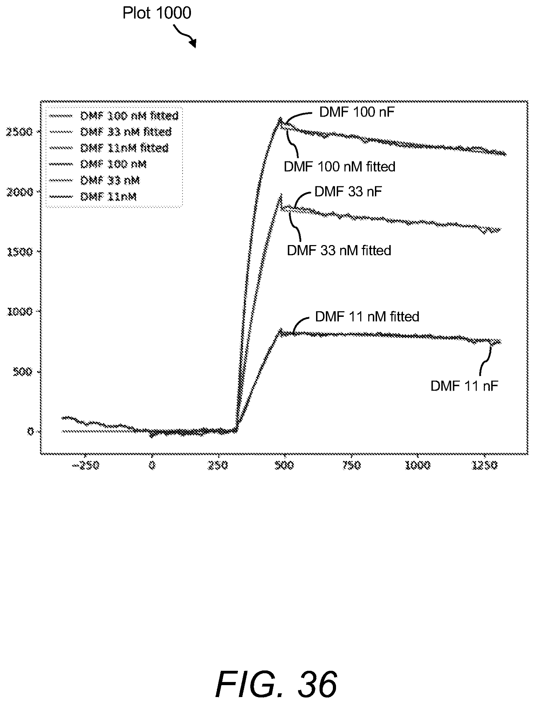

The results of this experiment are shown in FIG. 35 and FIG. 36. Referring now to FIG. 35, a plot 900 of the protein A/IgG experimental data from both the DMF instrument as well as the OpenSPR is shown, demonstrating an improvement in sensitivity and reduction in dispersion. The results of this test were imported into a standard analysis software, Tracedrawer, for kinetic analysis (see plot 900 of FIG. 35). This shows excellent fit parameters and resulted in a measured association constant (K_{ON}) of 1.36 x 10⁵ M⁻¹s⁻¹, a measured dissociation constant (K_{OFF}) of 1.17 x 10⁻⁴ s⁻¹ and a measured analyte affinity (K_{D}) of 0.86 nM which is expected based on the same assay performed on conventional equipment.

With reference now to FIG. 36 is a plot 1000 that shows the fitting of binding kinetics to the DMF Protein A data. Plot 1000 of FIG. 36 compares the results of the DMF-based test with an OpenSPR analysis device that uses a more conventional flow cell fluidic system. In comparison, the DMF device exhibits higher sensitivity and substantially reduced dispersion. Dispersion refers to the dispersing of a signal across the buffer-sample interface during a conventional flow cell-based SPR analysis. As the sample transits to and from the sensor surface, it is bordered typically with buffer, an interface across which the sample diffuses over. This causes the leading and trailing edges of the sample to have a sloped concentration gradient that is apparent in the signal. In the DMF device, due to the low sample volume and the ability to exchange the sample droplets without any exposure to a buffer front, the dispersion is not measurable. This lack of dispersion enables more accurate analysis of faster binding chemistries and improved kinetic fitting accuracy due to the lack of non-binding referred signal changes.

Following long-standing patent law convention, the terms "a," "an," and "the" refer to "one or more" when used in this application, including the claims. Thus, for example, a reference to "a subject" includes a plurality of subjects, unless the context clearly is to the contrary (e.g., a plurality of subjects), and so forth.

Throughout this specification and the claims, the terms "comprise," "comprises," and "comprising" are used in a non-exclusive sense, except where the context requires otherwise. Likewise, the term "include," and its grammatical variants are intended to be nonlimiting, such that recitation of items in a list is not to the exclusion of other like items that can be substituted or added to the listed items.

For the purposes of this specification and appended claims, unless otherwise indicated, all numbers expressing amounts, sizes, dimensions, proportions, shapes, formulations, parameters, percentages, quantities, characteristics, and other numerical values used in the specification and claims, are to be understood as being modified in all instances by the term "about" even though the term "about" may not expressly appear with the value, amount or range. Accordingly, unless indicated to the contrary, the numerical parameters set forth in the following specification and attached claims are not and need not be exact, but may be approximate and/or larger or smaller as desired, reflecting tolerances, conversion factors, rounding off, measurement error and the like, and other factors known to those of skill in the art depending on the desired properties sought to be obtained by the presently disclosed subject matter. For example, the term "about," when referring to a value can be meant to encompass variations of, in some embodiments ± 100%, in some embodiments ± 50%, in some embodiments ± 20%, in some embodiments ± 10%, in some embodiments ± 5%, in some embodiments ± 1%, in some embodiments ± 0.5%, and in some embodiments ± 0.1% from the specified amount, as such variations are appropriate to perform the disclosed methods or employ the disclosed compositions.

Further, the term "about" when used in connection with one or more numbers or numerical ranges, should be understood to refer to all such numbers, including all numbers in a range and modifies that range by extending the boundaries above and below the numerical values set forth. The recitation of numerical ranges by endpoints includes all numbers, e.g., whole integers, including fractions thereof, subsumed within that range (for example, the recitation of 1 to 5 includes 1, 2, 3, 4, and 5, as well as fractions thereof, e.g., 1.5, 2.25, 3.75, 4.1, and the like) and any range within that range.

Although the foregoing subject matter has been described in some detail by way of illustration and example for purposes of clarity of understanding, it will be understood by those skilled in the art that certain changes and modifications can be practiced within the scope of the appended claims.
Embodiments of the present invention are disclosed in the following clauses E1 to E86.
E1. A cartridge for use with an instrument to perform measurement of a fluid, comprising:
   a digital microfluidics (DMF) portion comprising a plurality of droplet actuators operative to perform droplet operations on a fluid droplet in the DMF portion; and
   a reaction portion comprising sensor media that is disposed in relation to the plurality of droplet actuators, wherein the plurality of droplet actuators are operative to induce movement of the fluid droplet relative to the sensor media while in contact with the sensor media.
E2. The cartridge of E 1, wherein the plurality of droplet actuators comprise a plurality of reaction electrodes.
E3. The cartridge of E 2, wherein the plurality of reaction electrodes perform droplet operations by electrowetting.
E4. The cartridge of E 3, wherein the sensor media comprises surface plasmon resonance (SPR) sensor media.
E5. The cartridge of E 4, wherein the SPR sensor media is functionalized with a capture molecule to which a target molecule of an analyte fluid binds to change an optical signal of the SPR sensor media.
E6. The cartridge of E 5, wherein the capture molecule comprises a ligand immobilized on the SPR sensor media that is sensitive to binding with the target molecule of the analyte fluid to change an optical property of the SPR sensor media resulting in the change of the optical signal of the SPR sensor media.
E7. The cartridge of E 6, wherein the change of the optical properties comprises a change in the optical signal resulting from light interacting with the SPR sensor media.
E8. The cartridge of E 7, further comprising:
   an SPR sensor surface disposed in the reaction portion and in relation to the plurality of reaction electrodes, wherein the SPR sensor media is disposed on the SPR sensor surface, and wherein the droplet is contactingly engageable with the SPR sensor surface by operation of the plurality of reaction electrodes.
E9. The cartridge of E 8, wherein the SPR sensor media comprises one of nanosized structures distributed on the sensor surface or a continuous film comprising nano-sized features.
E10. The cartridge of E 9, wherein the reaction portion comprises a first substrate and second substrate disposed in spaced-apart relation to define a reaction chamber therebetween.
E11. The cartridge of E 10, wherein the SPR sensor surface is disposed at the first substrate, and the plurality of reaction electrodes are disposed at the second substrate opposite the first substrate.
E12. The cartridge of E 10, wherein the SPR sensor surface is disposed at the first substrate, and the plurality of reaction electrodes are disposed at the first substrate.
E13. The cartridge of E 10, wherein the SPR sensor surface is disposed adjacent to a terminal portion of an optical member comprising at least one optical fiber, wherein the optical member extends away from one of the first substrate or the second substrate to dispose the SPR sensor surface within the reaction chamber.
E14. The cartridge of E 13, wherein the optical member comprises a first optical fiber on which the optical signal is transmitted from the SPR sensor surface.
E15. The cartridge of E 14, wherein the optical member comprises a second optical fiber on which light from an illumination source is provided to the SPR sensor surface.
E16. The cartridge of E 13, wherein the optical member is moveable relative to the first substrate to dispose the SPR sensor surface between an extended position in which the SPR sensor surface is disposed in the reaction chamber and a retracted position in which the SPR sensor surface is not disposed in the reaction chamber.
E17. The cartridge of E 13, wherein the reaction chamber contains a filler media.
E18. The cartridge of E 17, wherein the optical member is retractable to reduce contact between the SPR sensor surface and the filler media.
E19. The cartridge of E 9, wherein the SPR sensor surface is disposed between a first reaction electrode and a second reaction electrode.
E20. The cartridge of E 19, wherein the first reaction electrode and the second reaction electrode are alternately activated to induce oscillation of the droplet between the first reaction electrode and the second reaction electrode to induce the movement of the fluid droplet relative to the SPR sensor surface.
E21. The cartridge of E 20, wherein the oscillation of the droplet between the first reaction electrode and the second reaction electrode is linear.
E22. The cartridge of E 9, wherein the SPR sensor surface is disposed between three or more reaction electrodes, and wherein the three or more reaction electrodes are alternately activated to induce oscillation of the droplet between the three or more reaction electrodes to induce the movement of the fluid droplet relative to the SPR sensor surface.
E23. The cartridge of E 22, wherein the oscillation of the droplet between the three or more reaction electrodes is circular.
E24. The cartridge of E 7, wherein the sensor media comprises a plurality of sensor nanoparticles suspended in a sensor droplet disposed in the reaction portion.
E25. The cartridge of E 24, wherein the fluid droplet is merged with the sensor droplet to form a reacted droplet for measurement of the optical signal of the SPR sensor media in the reacted droplet.
E26. The cartridge of E 25, wherein the movement induced by the plurality of droplet actuators is operative to mix the reacted droplet.
E27. The cartridge of E 26, wherein each of the plurality of sensor nanoparticles is magnetically responsive.
E28. The cartridge of E 27, wherein each of the plurality of sensor nanoparticles comprise a magnetically responsive core.
E29. The cartridge of E 27, wherein each of the plurality of sensor nanoparticles comprise a magnetically responsive element tethered to the sensor nanoparticle.
E30. The cartridge of E 29, wherein the magnetically responsive element is physically or chemically coupled to the sensor nanoparticle.
E31. The cartridge of E 27, further comprising:
   a magnet that is selectively operable to act on the magnetically responsive sensor nanoparticles to immobilize the sensor nanoparticles in the reaction portion to dispose the plurality of nanoparticles in a restrained position relative to the magnet.
E32. The cartridge of E 31, wherein the plurality of droplet actuators are operative to move fluid away from the sensor nanoparticles when the magnetically responsive sensor nanoparticles are immobilized by the magnet in the restrained position and to move fluid into contact with the sensor nanoparticles when the magnetically responsive sensor nanoparticles are immobilized by the magnet in the restrained position.
E33. The cartridge of E 1, wherein the movement of the fluid droplet is at a rate greater than a sampling rate of an optical system measuring an optical signal of the sensor media.
E34. The cartridge of E 2, further comprising:
   a plurality of droplet operation electrodes in the DMF portion that are operative to supply fluid to the plurality of droplet actuators.
E35. The cartridge of E 34, further comprising:
   a reservoir electrode in the DMF portion to receive and maintain the fluid in the DMF portion.
E36. The cartridge of E 34, wherein the droplet operation comprises at least one of droplet merging, droplet splitting, droplet dispensing, or droplet diluting.
E37. The cartridge of E 1, wherein the fluid droplet comprises an analyte fluid droplet, and the movement of the analyte fluid droplet relative to the sensor media while in contact with the sensor media comprises an effective diffusion rate of the analyte fluid droplet relative to the sensor media.
E38. The cartridge of E 37, wherein the effective diffusion rate of the analyte fluid droplet is higher than a binding rate of an analyte relative to the SPR sensor.
E39. The cartridge of E 1, further comprising:
   an electrical contact in electrical communication with the plurality of droplet actuators, wherein the electrical contact is configured for interface with a controller for control of the plurality of droplet actuators.
E40. The cartridge of E 39, further comprising:
   a pluggable interface of the cartridge comprising the electrical contact, wherein the pluggable interface is physically and electrically engageable with an instrument to establish electrical communication between a controller of the instrument and the plurality of droplet actuators.
E41. The cartridge of E 1, wherein the reaction portion is substantially transparent to an illumination source incident on the reaction portion on at least one side of the reaction portion to facilitate real-time optical measurement of the sensor media in a reflectance mode.
E42. The cartridge of E 1, wherein the reaction portion is substantially transparent to an illumination source incident on the reaction portion on opposite sides of the reaction portion to facilitate real-time optical measurement of the sensor media in a transmission mode.
E43. The cartridge of E 7, wherein the fluid droplet comprises an analyte fluid droplet, the SPR sensor media is operable to detect analyte affinity of the analyte fluid droplet during the movement of the analyte fluid droplet relative to the sensor media, and wherein the analyte affinity is characterized by an analyte affinity value (K_{D}).
E44. The cartridge of E 43, wherein the K_{D} is determined based on an on-rate (K_{ON}) measured during an association phase of the analyte fluid at the SPR sensor and an off-rate (K_{OFF}) measured during a dissociation phase of the analyte fluid at the SPR sensor.
E45. A plasmon resonance (PR) system, comprising:
   a cartridge according to E 1;
   a PR instrument with which the cartridge is engageable, the PR instrument comprising:
      a controller in operative communication with electrical contacts of the cartridge for control of the plurality of droplet actuators; and
      an optical detection system operative to measure an optical signal of the sensor media.
E46. The PR system of E 45, wherein the optical detection system further comprises:
   an illumination source operative to direct light incident to the sensor media; and
   an optical measurement device that measures the optical signal of the sensor media.
E47. The PR system of E 45, wherein the fluid droplet comprises an analyte fluid droplet, and wherein the controller is operative to detect a target molecule in the analyte fluid droplet based on the optical signal of the sensor media in the presence of the analyte fluid droplet while in motion relative to the sensor media.
E48. The PR system of E 47, wherein the controller is operative to measure binding events of the target molecule in the analyte fluid droplet in real time based on the optical signal of the sensor media in while the analyte fluid droplet is in motion relative to the sensor media.
E49. The PR system of any E 48, wherein the controller is operative to determine a quantitative measurement of analyte affinity comprising an analyte affinity value (K_{D}). E50. The PR system of E 49, wherein the K_{D} is determined based on an on-rate (K_{ON}) measured during an association phase of the sensor media and an off-rate (K_{OFF}) measured during a dissociation phase of the sensor media.
E51. The PR system of E 50, wherein the fluid droplet in the reaction portion comprises an analyte fluid droplet during the association phase, and wherein the fluid in the reaction portion comprises a buffer solution fluid during the dissociation phase.
E52. A method of operation of a cartridge for measurement of an analyte fluid, comprising:
   contacting sensor media in a reaction portion of the cartridge with an analyte fluid droplet;
   inducing movement of the analyte fluid droplet with respect to the sensor media while maintaining the analyte fluid droplet in contact with the sensor media, wherein the inducing comprises operation of a plurality of droplet actuators disposed relative to the sensor media; and
   generating a first optical signal at the sensor media during the movement of the analyte fluid droplet relative to the sensor media.
E53. The method of E 52, wherein the first optical signal comprises an association signal corresponding to an association phase of the sensor media in the presence of the analyte fluid droplet.
E54. The method of E 53, further comprising:
   determining an on-rate (K_{ON}) of the analyte fluid droplet based on the association signal.
E55. The method of E 54, wherein the determining the K_{ON} comprises fitting an association curve to the association signal.
E56. The method of E 55, further comprising:
   moving the analyte fluid droplet from the reaction portion such that the analyte fluid droplet is no longer in contacting engagement with the sensor media;
   introducing a buffer solution fluid droplet to the reaction portion, wherein the buffer solution is in contacting engagement with the sensor media;
   inducing movement of the buffer solution fluid droplet with respect to the sensor media while maintaining the buffer solution fluid droplet in contact with the sensor media, wherein the inducing comprises operation of the plurality of droplet actuators disposed relative to the sensor media; and
   generating a second optical signal at the sensor media during the movement of the buffer solution fluid droplet relative to the sensor media.
E57. The method of E 56, wherein the second optical signal comprises a dissociation signal corresponding to a dissociation phase of the sensor media in the presence of the buffer solution fluid droplet.
E58. The method of E 57, further comprising:
   determining an off-rate (K_{OFF}) of the analyte fluid based on the dissociation signal.
E59. The method of E 58, wherein the determining the K_{OFF} comprises fitting a dissociation curve to the dissociation signal.
E60. The method of E 59, further comprising:
   calculating an analyte affinity value (K_{D}) based on the K_{ON} and the K_{OFF}.
E61. The method of E 60, wherein K_{D} is the quotient of K_{ON} and K_{OFF}.
E62. The method of E 52, wherein the sensor media comprises a plurality of sensor nanoparticles disposed in the reaction portion.
E63. The method of E 62, wherein the plurality of sensor nanoparticles are disposed in a sensor droplet, and the method further comprises:
   merging the analyte fluid droplet and the sensor to form a reacted droplet for measurement of the optical signal of the sensor media in the reacted droplet.
E64. The method of E 62, wherein each of the plurality of sensor nanoparticles is magnetically responsive.
E65. The method of E 62, further comprising:
   activating a magnet to dispose the nanoparticles in a restrained position relative to the magnet to immobilize the sensor nanoparticles in the reaction portion.
E66. The method of E 65, wherein the nanoparticles are maintained in the restrained position relative to the magnet during moving of a droplet relative to the reaction portion.
E67. The method of E 52, wherein the sensor media is disposed adjacent to a terminal portion of a moveable member, and the method further comprises:
   moving the moveable member relative to a reaction chamber defined in the reaction portion between an extended position and a retracted position, wherein the sensor media is disposed in the reaction chamber in the extended position and is removed from the reaction chamber in the retracted position.
E68. The method of E 67, wherein the reaction chamber comprises a filler media, and the method further comprises:
   retracting the moveable member to the retracted position;
   introducing a fluid droplet to the reaction portion after the retracting to displace the filler media from an area adjacent to the plurality of droplet actuators; and
   advancing the moveable member after the introducing to the extended position to dispose the sensor media in the fluid droplet.
E69. The method of E 52, further comprising:
   engaging the cartridge with an instrument.
E70. The method of E 69, further comprising:
   measuring a signal from the sensor media while the fluid droplet is moved relative to the sensor media while maintaining the fluid droplet in contact with the sensor media.
E71. The method of E 70, wherein the sensor media comprises SPR sensor media, and the signal comprises an optical signal of the SPR sensor media.
E72. The method of E 71, further comprising:
   providing light from a light source of the instrument incident to the SPR sensor media; and
   wherein the measuring comprises measuring the optical signal of the SPR sensor media at an optical measurement device of the instrument.
E73. The method of E 69, wherein the engaging further comprises:
   establishing electrical communication between a controller of the instrument and the plurality of droplet actuators of the DMF portion;
   controlling the plurality of droplet actuators of the DMF portion; and
   wherein the inducing movement of the fluid is in response to the controlling of the plurality of droplet actuators of the DMF portion.
E74. The method of E 73, wherein in a first period the fluid comprises a buffer fluid droplet, and the measuring comprises recording a baseline optical signal as the buffer fluid is moved relative to the sensor media while maintaining contact with the sensor media.
E75. The method of E 74, wherein the method further comprises:
   introducing an analyte fluid droplet to the reaction portion in a second period, wherein the measuring comprises capturing an association signal corresponding to an association phase of the analyte fluid droplet in the second period.
E76. The method of E 75, wherein an effective diffusion rate of the analyte fluid droplet relative to the sensor media is higher than a binding rate of the analyte fluid droplet relative to the sensor media.
E77. The method of E 76, further comprising:
   determining an on-rate (K_{ON}) of the analyte fluid droplet based on the association signal.
E78. The method of E 77, wherein the determining the K_{ON} comprises fitting an association curve to the association signal.
E79. The method of E 78, further comprising:
   moving the analyte fluid droplet away from the sensor media; and
   introducing a buffer fluid droplet to the reaction portion in a third period, wherein the measuring comprises capturing a dissociation signal corresponding to a dissociation phase of the analyte in the third period.
E80. The method of E 79, further comprising:
   determining an off-rate (K_{OFF}) of the analyte fluid based on the dissociation signal.
E81. The method of E 80, wherein the determining the K_{OFF} comprises fitting a dissociation curve to the dissociation signal.
E82. The method of E 81, further comprising:
   calculating an analyte affinity value (K_{D}) based on the K_{ON} and the K_{OFF}.
E83. The method of E 82, wherein K_{D} is the quotient of K_{ON} and K_{OFF}.
E84. The method of E 83, further comprising:
   supplying, in a fourth period, a regeneration buffer solution fluid droplet to the reaction portion; and
   contacting the regeneration buffer solution fluid droplet with the sensor media to regenerate the sensor media.
E85. The method of E 84, further comprising:
   functionalizing the sensor media by contacting a functionalization fluid droplet comprising ligands to bind the ligands to the sensor media.
E86. The method of E 85, further comprising:
   activating the sensor media by contacting an activation fluid droplet with the sensor media prior to the functionalizing of the sensor media.

## Claims

1. A method of operation of a cartridge for measurement of an analyte fluid, comprising:
contacting sensor media in a reaction portion of the cartridge with a fluid droplet;
inducing movement of the fluid droplet with respect to the sensor media while maintaining the fluid droplet in contact with the sensor media, wherein the inducing comprises operating a plurality of droplet actuators disposed relative to the sensor media; and
providing light to the sensor media during the movement of the fluid droplet relative to the sensor media.

2. The method of claim 1, further comprising measuring a signal from the sensor media while the fluid droplet is moved relative to the sensor media while maintaining the fluid droplet in contact with the sensor media.

3. The method of claim 2, wherein the sensor media comprises a plurality of sensor nanoparticles disposed in the reaction portion.

4. The method of claim 2, wherein the sensor media comprises surface plasmon resonance (SPR) sensor media, and the signal comprises an optical signal of the SPR sensor media.

5. The method of claim 4, further comprising: engaging the cartridge with an instrument;
wherein the engaging comprises:
establishing electrical communication between a controller of the instrument and the plurality of droplet actuators;
controlling the plurality of droplet actuators; and
wherein the inducing movement of the fluid droplet is in response to the controlling of the plurality of droplet actuators.

6. The method of claim 5, further comprising:
providing the light from a light source of the instrument incident to the SPR sensor media; and
wherein the measuring comprises measuring the optical signal of the SPR sensor media at an optical measurement device of the instrument.

7. The method of claim 4, wherein in a first period the fluid droplet comprises a buffer fluid droplet, and the optical signal is a baseline optical signal as the buffer fluid droplet is moved relative to the SPR sensor media while maintaining contact with the SPR sensor media.

8. The method of claim 7, wherein in a second period the fluid droplet comprises an analyte fluid droplet, and the optical signal is an association signal corresponding to an association phase of the analyte fluid droplet in the second period.

9. The method of claim 8, wherein an effective diffusion rate of the analyte fluid droplet relative to the SPR sensor media is higher than a binding rate of the analyte fluid droplet relative to the SPR sensor media.

10. The method of claim 9, further comprising:
determining an on-rate (K_{ON}) of the analyte fluid droplet based on the association signal.

11. The method of claim 10, wherein in a third period the fluid droplet comprises a second buffer fluid droplet, and the optical signal is a dissociation signal corresponding to a dissociation phase of the analyte fluid droplet in the third period.

12. The method of claim 11, further comprising: determining an off-rate (K_{OFF}) of the analyte fluid droplet based on the dissociation signal.

13. The method of claim 12, further comprising:
supplying, in a fourth period, a regeneration buffer solution fluid droplet to the reaction portion; and
contacting the regeneration buffer solution fluid droplet with the SPR sensor media to regenerate the SPR sensor media.

14. The method of claim 13, further comprising:
functionalizing the SPR sensor media by contacting the SPR sensor media with a functionalization fluid droplet comprising ligands to bind the ligands to the SPR sensor media.

15. The method of claim 14, further comprising:
activating the SPR sensor media by contacting an activation fluid droplet with the SPR sensor media prior to the functionalizing of the SPR sensor media.
